# EUROPEAN PATENT SPECIFICATION

(11) **EP 1 874 345 B1**
(45) Date of publication and mention of the grant of the patent: **15.08.2012**
(21) Application number: 06758610.7
(22) Date of filing: 25.04.2006
(51) Int. Cl.: A61K 39/395, A61K 39/385, A61K 39/39, C07K 14/16, C12N 7/04, C12N 15/86

(54) **IMMUNOSTIMULATORY COMPOSITIONS**
IMMUNSTIMULIERENDE ZUSAMMENSETZUNGEN
COMPOSITIONS IMMUNOSTIMULANTES

(30) Priority: 25.04.2005 US 674753 P
(43) Date of publication of application: 09.01.2008
(73) Proprietor: 3M Innovative Properties Company, St. Paul, MN 55133-3427 (US)
(72) Inventor: GRIESGRABER, George W., Saint Paul, MN 55133-3427 (US); KIEPER, William C., Stillwater, MN 55082 (US); MENDOZA, James D., Saint Paul, MN 55133-3427 (US); GRAM, Christopher D., Saint Paul, MN 55133-3427 (US)
(74) Representative: Vossius & Partner
(86) International application number: PCT/US2006/015772
(87) International publication number: WO 2006/116475

(56) References cited:
- WO-A-2004/032829
- WO-A-2004/060319
- WO-A-2005/020999
- CA-A1- 2 521 529
- US-A1- 2004 091 491
- US-A1- 2004 091 491
- US-A1- 2004 202 720
- HARRISON C J ET AL: "Reduction of recurrent HSV disease using imiquimod alone or combined with a glycoprotein vaccine" VACCINE, BUTTERWORTH SCIENTIFIC. GUILDFORD, GB, vol. 19, no. 13-14, 1 January 2001 (2001-01-01), pages 1820-1826, XP002249629 ISSN: 0264-410X
- WU J J ET AL: "Resiquimod: a new immune response modifier with potential as a vaccine adjuvant for Th1 immune responses" ANTIVIRAL RESEARCH, ELSEVIER SCIENCE BV., AMSTERDAM, NL, vol. 64, no. 2, 1 November 2004 (2004-11-01), pages 79-83, XP004605743 ISSN: 0166-3542
- BERNSTEIN D I ET AL: "ADJUVANT EFFECTS OF IMIQUIMOD ON A HERPES SIMPLEX VIRUS TYPE 2 GLYCOPROTEIN VACCINE IN GUINEA PIGS" JOURNAL OF INFECTIOUS DISEASES, CHICAGO, IL, vol. 167, no. 3, 1 March 1993 (1993-03-01), pages 731-735, XP001037924 ISSN: 0022-1899
- EVANS T G ET AL: "QS-21 promotes an adjuvant effect allowing for reduced antigen dose during HIV-1 envelope subunit immmunization in humans" VACCINE, BUTTERWORTH SCIENTIFIC. GUILDFORD, GB, vol. 19, no. 15-16, 28 February 2001 (2001-02-28), pages 2080-2091, XP004316949 ISSN: 0264-410X

## Description

### Background

There has been a major effort in recent years, with significant success, to discover new drug compounds that act by stimulating certain key aspects of the immune system, as well as by suppressing certain other aspects (see, e.g., U.S. Pat. Nos. 6,039,969 and 6,200,592). These compounds, referred to herein as immune response modifiers (IRMs), appear to act through basic immune system mechanisms known as Toll-like receptors (TLRs) to induce selected cytokine biosynthesis, induction of co-stimulatory molecules, and increased antigen-presenting capacity. They may be useful for treating a wide variety of diseases and conditions. For example, certain IRMs may be useful for treating viral diseases (e.g., human papilloma virus, hepatitis, herpes), neoplasias (e.g., basal cell carcinoma, squamous cell carcinoma, actinic keratosis, melanoma), and T_{H}2-mediated diseases (e.g., asthma, allergic rhinitis, atopic dermatitis), auto-immune diseases (e.g., multiple sclerosis), and are also useful as vaccine adjuvants.

Many of the IRM compounds are small organic molecule imidazoquinoline amine derivatives (see, e.g., U.S. Pat. No. 4,689,338), but a number of other compound classes are known as well (see, e.g., U.S. Pat. Nos. 5,446,153;.6,194,425; and 6,110,929) and more are still being discovered. Other IRMs have higher molecular weights, such as oligonucleotides, including CpGs (see, e.g., U.S. Pat. No. 6,194,388). In WO 2004/032829 it has been found that IRMs may be more effective in stimulating an immune response when chemically or physically paired with antigen.

In view of the great therapeutic potential for IRMs, and despite the important work that has already been done, there is a substantial ongoing need to expand their uses and therapeutic benefits.

### Summary

It has been found that conjugating an IRM to an antigenic peptide generates more than one immunoreactive fraction, each of which contains material that is more potent at generating an immune response than a simple mixture of IRM and the antigenic peptide. Moreover, the immunoreactive fractions are separable, providing the possibility of at least partially purifying the fractions and using one or more of the partially purified fractions to produce an immunostimulatory composition.
Accordingly, the present invention provides an immunostimulatory composition.

The immunostimulatory composition includes an at least partially purified antigenic aggregate that includes an IRM moiety and at least two antigenic peptides in which at least one antigenic peptide is covalently coupled to the IRM moiety. In some embodiments, the immunostimulatory composition may be a vaccine.

In another aspect, the present invention also provides a method of making an immunostimulatory composition that includes providing a mixture of IRM compound and antigenic peptides; causing at least one IRM moiety to covalently couple to an antigenic peptide to form at least two immunostimulatory fractions from the mixture; and separating at least a portion of one immunostimulatory fraction from at least a portion of the second immunostimulatory fraction.

In some embodiments, one immunostimulatory fraction may contain antigenic aggregates.

In another aspect, this description provides a method of inducing an antigen-specific immune response that, in general, includes contacting an immune cell with an immunostimulatory composition that includes an at least partially purified IRM/antigen conjugate that includes at least one antigenic peptide covalently coupled to an IRM moiety. In other embodiments, the immunostimulatory composition may include an antigenic aggregate that includes an IRM/antigen conjugate in noncovalent association with at least one other antigenic peptide, which may be a free antigenic peptide or an antigenic peptide moiety of a second IRM/antigen conjugate.

In yet another aspect, the present description provides a method of treating a condition that, in general, includes administering to a subject an immunostimulatory composition that includes an at least partially purified IRM/antigen conjugate in an amount effective to provide prophylactic or therapeutic treatment of the condition.

In other embodiments, the immunostimulatory composition may include an antigenic aggregate that includes an IRM/antigen conjugate in noncovalent association with at least one other antigenic peptide, which may be a free antigenic peptide or an antigenic peptide moiety of a second IRM/antigen conjugate.

Various other features and advantages of the present invention should become readily apparent with reference to the following detailed description, examples, claims and appended drawings. In several places throughout the specification, guidance is provided through lists of examples. In each instance, the recited list serves only as a representative group and should not be interpreted as an exclusive list The invention is also defined by the claims.

### Brief Description of the Drawings

Fig. 1 is a bar graph showing immunogenicity data for various pre-HPLC and post-HPLC samples collected from mixtures of photoreactive IRM and/or antigen with or without exposure to UV.
Fig. 2 is an HPLC chromatogram of an antigen alone after exposure to UV.
Fig. 3 is an HPLC chromatogram of reaction products resulting from a 4:1 molar ratio mixture of photoreactive IRM and antigen after exposure to UV.
Fig. 4 is an HPLC chromatogram of reaction products resulting from an 8:1 molar ratio mixture of photoreactive IRM and antigen after exposure to UV.
Fig. 5 is an HPLC chromatogram of a second antigen alone after exposure to UV.
Fig. 6 is an HPLC chromatogram of reaction products resulting from of a 4:1 molar ratio mixture of photoreactive IRM and the second antigen after exposure to UV.
Fig. 7 is an HPLC chromatogram of a third antigen alone.
Fig. 8 is an HPLC chromatogram of reaction products resulting from a 4:1 molar ratio mixture of a photoreactive IRM and the third antigen after exposure to UV.
Fig. 9 is an HPLC chromatogram of reaction products resulting from conjugating an antigen and IRM2.
Fig. 10 is an HPLC chromatogram of reaction products resulting from conjugating an antigen and IRM3.
Fig. 11 is an HPLC chromatogram of reaction products resulting from conjugating an antigen and L-cysteine.
Fig. 12 is a bar graph showing antibody titers induced by IRM-HEL conjugation products.
Fig. 13 is a bar graph showing expansion of antigen-specific lymphocytes after immunization with IRM/antigen conjugates.
Fig. 14 is an HPLC chromatogram of an antigen alone.
Fig. 16 is an HPLC chromatogram of reaction products resulting from of a 20:1 molar ratio mixture of Sulfo-SMCC and antigen before mixture with IRM2.

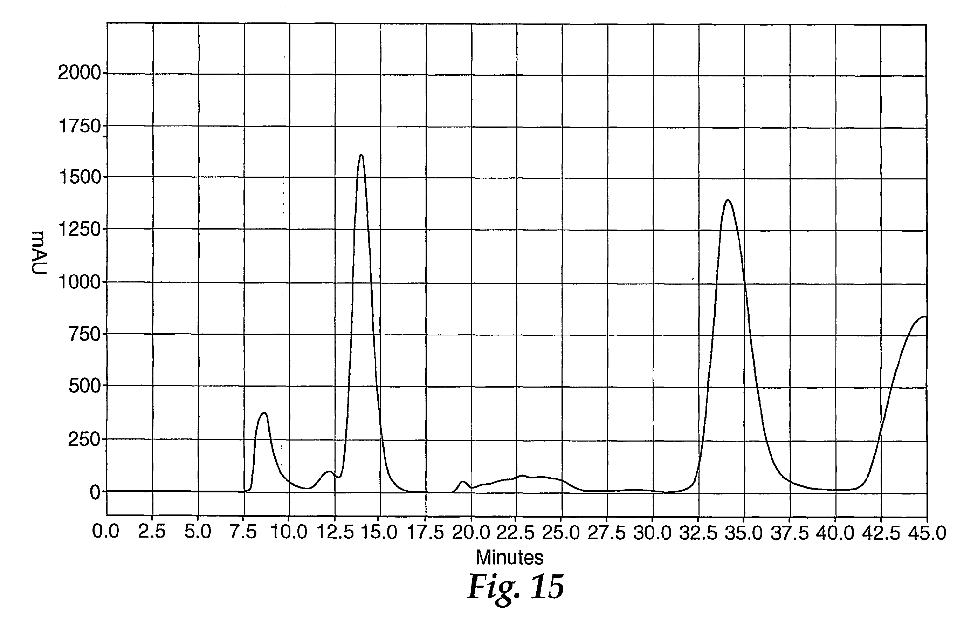

Fig. 15 is an HPLC chromatogram of reaction products resulting from of a 40:1 molar ratio mixture of Sulfo-SMCC and antigen before mixture with IRM2.

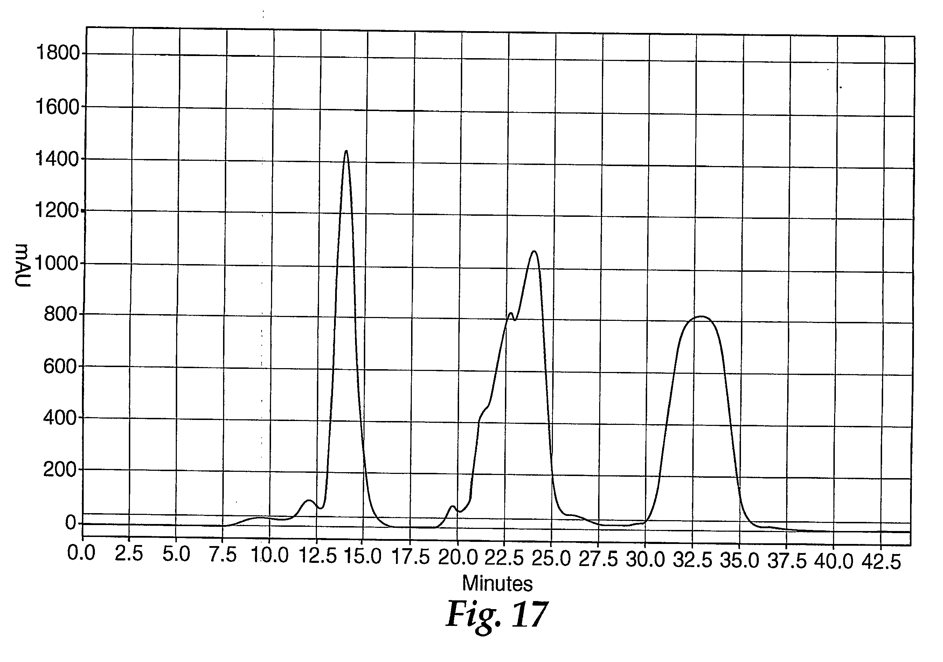

Fig. 17 is an HPLC chromatogram of reaction products resulting from of a 20:1 molar ratio mixture of Sulfo-SMCC and antigen before mixture with IRM3.

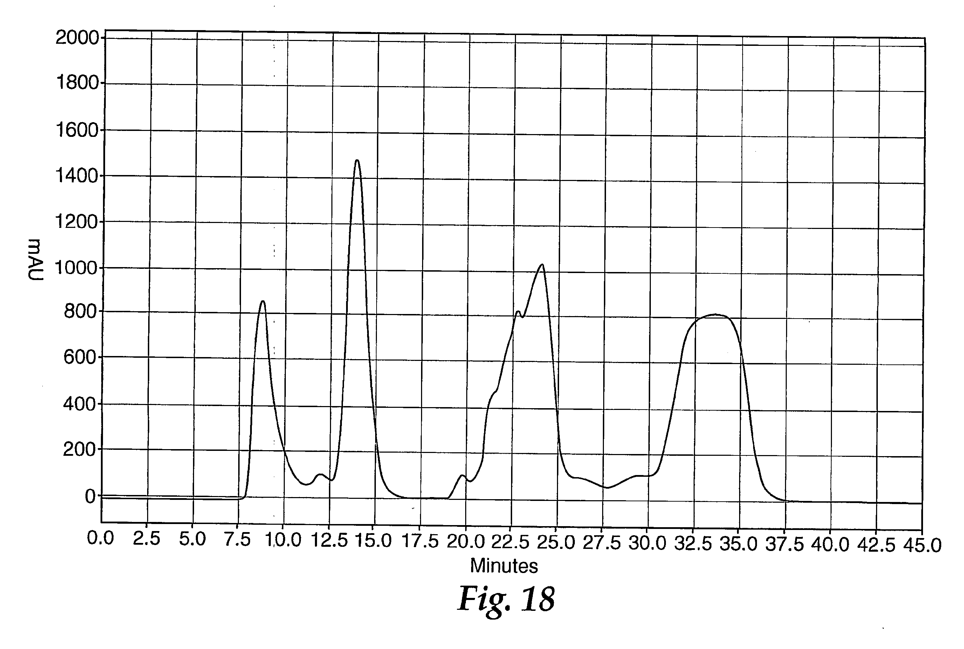

Fig. 18 is an HPLC chromatogram of reaction products resulting from of a 40:1 molar ratio mixture of Sulfo-SMCC and antigen before mixture with IRM3.

### Detailed Description of Illustrative Embodiments of the Invention

The present invention generally provides preparations effective for generating an immune response and methods of generating an immune response including, in some cases, methods for treating certain conditions. Generally, the preparations include a conjugate of antigenic peptide and an immune response modifier (IRM), the preparation includes an aggregation of antigenic peptides.

It has been found that an IRM/antigen conjugate preparation can be more effective at raising an immune response than the antigen alone, IRM alone, or a mixture of IRM and the antigen. Thus, an immunostimulatory composition of the invention may result in increasing the immune response to an antigenic peptide that is part of the immunostimulatory composition. Increasing the immunogenicity of an antigenic peptide in an immunostimulatory composition can provide one or more benefits such as, for example, fewer administrations of the immunostimulatory composition to achieve a desired result, improving or establishing the efficacy of a immunostimulatory composition, faster or more complete treatment, reduced side effects associated with the immunostimulatory composition, or lower costs.

For example, certain vaccines include multiple antigenic peptides, some of which (e.g., toxoids) may cause undesirable side effects such as, for example, pain, swelling, tenderness, and the like. Practicing the invention may increase the immune response to a particular antigenic peptide of an immunostimulatory composition (e.g., a vaccine toxoid) sufficiently so that less of the particular antigenic peptide may be needed to provide the desired level of immune response, thereby reducing or even eliminating undesirable side effects of the antigenic peptide.

Requiring less of each antigenic peptide of the immunostimulatory composition to achieve a desired immune response can result in (a) lower costs to produce the immunostimulatory composition, such as when a particular antigenic peptide is costly to, for example, obtain or formulate, or (b) the ability to distribute the immunostimulatory composition more broadly such as, for example, when a particular antigenic peptide of the immunostimulatory composition is rare or is prohibitively costly.

Also, incorporating an antigenic peptide into an immunostimulatory composition of the invention may improve or help establish the efficacy of the antigenic peptide for providing effective treatment of a condition. Thus, an antigenic peptide that in other immunostimulatory compositions fails to induce a sufficient immune response to be considered effective for treating a particular condition may, when incorporated into an immunostimulatory composition of the invention, generate a sufficient immune response to prove effective for treating the condition.

While both the partially purified monomeric conjugate and the partially purified aggregate are more potent inducers of antigen-specific immune responses than an unconjugated mixture of antigen and IRM, the aggregate is generally more potent than the monomeric conjugate. Thus, one may be able to control the extent to which an antigen-specific immune response is increased by controlling the relative amounts of aggregate and monomeric conjugate in the immunostimulatory composition.

Thus, in one aspect, the present invention provides an immunostimulatory composition. Generally, the immunostimulatory composition includes an at least partially purified antigenic conjugate that includes at least one antigenic peptide covalently coupled to an IRM moiety to form an IRM/antigen conjugate. In other embodiments, the immunostimulatory composition includes an aggregate in which an IRM/antigen conjugate molecule associates noncovalently with at least one additional antigenic peptide. The additional antigenic peptide may be another IRM/conjugate molecule or unconjugated, free antigenic peptide. In some embodiments, the immunostimulatory composition can include a substantially homogeneous set of antigenic peptides, while in other embodiments the immunostimulatory composition can include at least two different antigenic peptides.

In another aspect, the invention provides methods of making an immunostimulatory composition. Generally, the methods include providing a mixture of IRM molecules and antigenic peptide molecules, causing antigenic aggregates to form, thereby also defining a non-aggregated portion of the mixture, and separating at least a portion of the antigenic aggregates from at least a portion of the non-aggregated portion of the mixture.

In yet another aspect, this description provides methods of using the immunostimulatory compositions. Generally, the methods include using an immunostimulatory composition that includes an at least partially purified aggregate to generate an antigen-specific immune response - i.e., an immune response directed against an antigenic peptide included in the immunostimulatory composition. In some cases, this permits the generation of an immune response that is directed against cells affected by certain types of conditions (e.g., tumor cells or cells infected with a virus). Such an immune response can provide directed immunological treatment of conditions such as, for example, cancers, viral infections, bacterial infections, etc.

As used herein, the following terms shall have the indicated meanings:
"Ameliorate" refers to any reduction in the extent, severity, frequency, and/or likelihood of a symptom or clinical sign characteristic of a particular condition.
"Antigen" and variations thereof refers to any substance that is capable of being the target of an immune response. An antigen may be the target of, for example, a cell-mediated and/or humoral immune response raised by a subject organism. Alternatively, an antigen may be the target of a cellular immune response (e.g., immune cell maturation, production of cytokines, production of antibodies, etc.) when contacted with immune cells.
"Moiety" and variations thereof refer to a portion of a chemical compound that exhibits a particular character such as, for example, a particular biological (e.g., immunomodulation) or chemical function (e.g., photoreactivity). In the context of compositions according to the present invention, the term moiety may be used to refer to an uncoupled chemical compound or portion thereof that, when coupled to another chemical compound or portion thereof, can be a moiety of the resulting coupled immunostimulatory composition.
"Peptide" refers to a sequence of amino acid residues without regard to the length of the sequence, whether naturally occurring or synthetic. Therefore, the term "peptide" refers to any amino acid sequence having at least two amino acids and includes full-length proteins and, as the case may be, polyproteins.
"Treat" or variations thereof refer to reducing, limiting progression, ameliorating, or resolving, to any extent, the symptoms or signs related to a condition.

As used herein, "a," "an," "the," "at least one," and "one or more" are used interchangeably. Thus, for example, an IRM/antigenic peptide aggregate comprising "an" antigenic peptide can be interpreted to mean that the IRM/antigenic peptide aggregate includes at least one antigenic peptide.

Unless otherwise indicated, reference to a compound can include the compound in any pharmaceutically acceptable form, including any isomer (e.g., diastereomer or enantiomer), salt, solvate, polymorph, and the like. In particular, if a compound is optically active, reference to the compound can include each of the compound's enantiomers as well as racemic mixtures of the enantiomers.

Also herein, the recitations of numerical ranges by endpoints include all numbers subsumed within that range (e.g., 1 to 5 includes 1, 1.5, 2, 2.75, 3, 3.80, 4, 5, etc.).

As noted above, IRM compounds are known to induce selected cytokine biosynthesis, induce co-stimulatory molecules, and increase antigen-presenting capacity. As a result, IRM compounds may be useful for treating a wide variety of diseases and conditions. Moreover, the ability of IRM compounds to modulate an immune response may be increased by co-presenting an antigen and the IRM compound to antigen presenting cells (APCs), as described in United States Patent Publication No. US2004/0091491.

One manner of co-presenting an IRM and an antigen is to first covalently couple the IRM and antigen to form an IRM-antigen conjugate. One way that this can be done uses an IRM compound that possesses a photoreactive moiety that becomes reactive when exposed to UV light. When the photoreactive IRM is mixed with a protein in solution and exposed to UV light, the result is a covalent linkage of at least one IRM and the antigenic protein. This technique has been used to generate IRM/antigen conjugates using many different proteins. When the conjugates are administered in vivo, they can generate a dramatically more potent T cell response than when the IRM and antigen are administered together as a non-conjugated mixture.

It has been found that the technique just described produces more than one immunoreactive fraction, each of which contains material that is more potent at generating an immune response than a simple mixture of IRM and antigen. One fraction includes a single protein molecule covalently coupled to one or more IRM moieties, referred to herein as a "monomeric conjugate." A second fraction includes a large molecular weight protein species, referred to herein as an "antigenic aggregate" or, simply, an "aggregate."

An antigenic aggregate includes at least one IRM/antigen conjugate that is in noncovalent association with additional peptides or the peptide moieties. Thus, an aggregate may include a plurality of IRM/antigen conjugates and/or unconjugated (or free) antigenic peptide. In all cases, however, an aggregate includes at least one IRM/antigen conjugate and so necessarily includes at least one IRM moiety covalently coupled to an antigenic peptide. Because a single IRM/antigen conjugate may include a plurality of IRM moieties, and because an aggregate may include a plurality of IRM/antigen conjugates, an aggregate may include many IRM moieties.

The fractions containing monomeric conjugate may be separated from the fractions containing aggregate on the basis of at least one difference in a chemical or physical property of the conjugation reaction products in the fraction. The result of such a separation is a set of fractions that are at least partially purified. As used herein, "at least partially purified" refers to immunostimulatory preparations in which the presence of a particular component (i.e., IRM/antigenic peptide aggregate) is enriched, to any degree, relative to the unpurified starting material. In some embodiments, "at least partially purified" may be characterized with respect to the total protein content (i.e., photoreacted protein + free protein) of the unpurified starting material. In certain embodiments, "at least partially purified" may be characterized with respect to the photoreacted protein content of the unpurified starting material.

As one example, conjugation of IRM to an antigenic peptide may yield a mixture of antigenic aggregate, monomeric conjugate, and free protein in which, for example, approximately 10% of the protein is in the aggregate form, 85% of the protein is in the monomeric conjugate form, and 5% of the protein remains free. Performing separation may produce a first fraction that contains, for example, half of the aggregate and a second fraction that contains, for example, the remaining protein. In this example, the first fraction may be considered an at least partially purified sample of aggregate because the relative amount of aggregate in the fraction has increased from the amount in the starting material - i.e., to nearly homogeneous from approximately 10%. The second fraction in this example may be considered an at least partially purified sample of the monomeric conjugate because it has been enriched for the monomeric conjugate as a result of the removal of some of the aggregate into the first fraction.

The conjugation reaction products may be separated on the basis of any suitable chemical or physical characteristic such as, for example, molecular weight, charge, size, etc. Techniques for such separations are well known and include, for example, chromatography (including, for example, high performance liquid chromatography - HPLC), electrophoresis, and dialysis. As used herein, "unpurified starting material" refers to the material to which a separation technique such as those just described is applied, regardless of whether the material may be the output of a previously performed separation technique such as, for example, dialysis to remove unreacted IRM, a solvent, or other non-proteinaceous component of the conjugation reaction mixture.

A degree of purification may be stated in any suitable terms such as, for example, an increase in the concentration of the purified component of the preparation, an increase in the molecular ratio or weight/weight ratio of the purified component compared to a second component, etc.

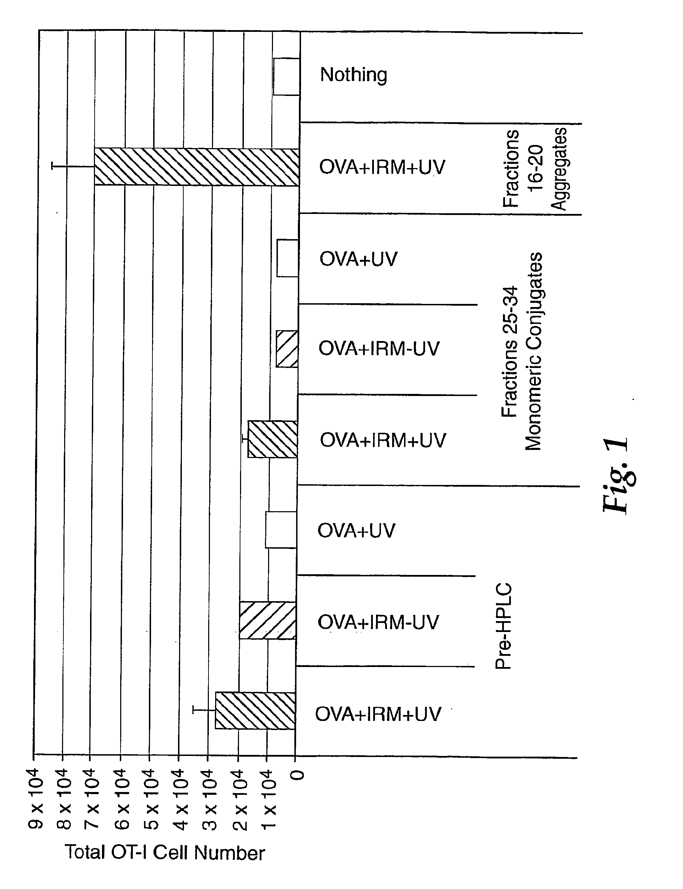

Once the fractions are separated (i.e., at least partially purified), each fraction can be evaluated for its ability to induce an immune response. Figure 1 compares the ability of various fractions of conjugation reaction products to induce an immune response. The monomeric IRM/antigen conjugate induces a greater immune response than either of (a) an unconjugated mixture of IRM and antigen, or (b) antigen exposed to UV light. The aggregate induces an immune response that exceeds even the monomeric conjugate.

Unless otherwise indicated, the term "immunostimulatory composition" refers herein to a composition that includes an at least partially purified antigenic aggregate. Unless otherwise indicated, IRM compounds and antigens characterized as suitable for use in preparing an immunostimulatory composition of the invention may be useful for preparing either a monomeric conjugate or an antigenic aggregate.

In one embodiment, an immunostimulatory composition includes an at least partially purified antigenic aggregate that includes at least one IRM moiety and at least two antigenic peptides in which at least one antigenic peptide is covalently coupled to the IRM moiety.

An antigenic peptide may be, or be derived from, any suitable antigen such as, for example, a microbial antigen, or a tumor antigen. The form of the antigenic portion may be a protein, an antigenic fragment of a protein, a lipoprotein, a glycoprotein, or, in certain cases, a polyprotein.

A microbial antigen as used herein is an antigen of, for example, a virus, a bacterium, a parasite, or a fungus. Thus, a microbial antigen may be a natural, full-length microbial protein, a polyprotein, or a fragment or a derivative of a natural microbial protein. Alternatively, a microbial antigen may be a synthetic compound that is identical to or sufficiently similar to a natural microbial antigen to induce an immune response (humoral and/or cellular) against the microbial antigen. Such antigens are used routinely in the art and are well known to those of ordinary skill in the art.

The antigenic peptide may be, or be derived from, a viral antigen. A viral antigen capable of eliciting an immune response against a human pathogen may be, or derived from, an antigen of, for example, HIV-1 (such as, for example, tat, nef, gp120, gp160, gp40, p24, gag, pol, env, vif, vpr, vpu, rev); human herpes viruses (such as, for example, gH, gL, gM, gB, gC, gK, gE, gD, or Immediate Early protein such as ICP27, ICP47, ICP 4, or ICP36 from HSV1 or HSV2); cytomegalovirus, especially Human, (such as, for example, gB); Epstein Barr virus (such as, for example, gp350); Varicella Zoster Virus (such as, for example, gpI, gpII, gpIII, or IE63); a hepatitis virus such as hepatitis B virus (e.g., PreS1, PreS2, Hepatitis B Surface antigen, Hepatitis B core antigen, or pol), hepatitis C virus, or hepatitis E virus; paramyxoviruses (e.g., Respiratory Syncytial virus (such as F and G proteins), parainfluenza virus, measles virus, mumps virus); human papilloma viruses (such as, for example, HPV6, 11, 16, 18, eg L1, L2, E1, E2, E3, E4, E5, E6, E7, E8); flaviviruses (e.g., Yellow Fever Virus, Dengue Virus, Tick-borne encephalitis virus, Japanese Encephalitis Virus); rhinoviruses, coronaviruses (e.g., SARS); or Influenza virus cells, such as HA, NP, NA, or M protein); or an antigenic fragment or, where appropriate, polyprotein of any of the foregoing.

Bacterial antigens suitable for use as an antigenic peptide, or from which an antigenic peptide may be derived include, for example, an iron-regulated outer membrane protein, (IROMP), an outer membrane protein (OMP), or an A-protein of *Aeromonis salmonicida,* which causes furunculosis; p57protein of *Renibacterium salmoninarum,* which causes bacterial kidney disease (BKD); major surface associated antigen (msa), a surface expressed cytotoxin (mpr), a surface expressed hemolysin (ish), or a flagellar antigen of Yersiniosis; an extracellular protein (ECP), an iron-regulated outer membrane protein (IROMP), or a structural protein of Pasteurellosis; an OMP or a flagellar protein of *Vibrosis anguillarum* or *V. ordalii;* a flagellar protein, an OMP protein, aroA, or purA of *Edwardsiellosis ictaluri* or *E. tarda;* a surface antigen of Ichthyophthirius; a structural or regulatory protein of *Cytophaga columnari;* or a structural or regulatory protein of Rickettsia; or an antigenic fragment of any of the foregoing.

Parasitic antigens suitable for use as an antigenic peptide, or from which an antigenic peptide may be derived include, for example, a surface antigen of Ichthyophthirius; an antigen from *Plasmodia falciparum* (e.g., RTS or a hybrid thereof such as RTS,S, TRAP, MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, Pfs27/25, Pfs16, Pfs48/45, or Pfs 230); or an antigenic fragment thereof.

A tumor antigen may be a compound, such as a peptide or protein, associated with a tumor or cancer cell surface that is capable of provoking an immune response when expressed on the surface of an antigen presenting cell in the context of an MHC molecule. A tumor antigen can be prepared from cancer cells by, for example, preparing crude extracts of cancer cells, for example, as described in Cohen, et al., 1994, Cancer Research, 54:1055 and partially purifying the antigens. Alternatively, a tumor antigen may be obtained by recombinantly expressing the antigen from a suitable vector in a suitable host, or by *de novo* synthesis of known antigens. Tumor antigens include but are not limited to full-length proteins or merely an immunogenic portion of a full-length tumor antigen protein. Such antigens can be isolated or prepared recombinantly or by any other means known in the art.

The terms "tumor antigen" and "cancer antigen" may be used interchangeably and refer to antigens that are differentially expressed by cancer cells and can therefore be exploited to generate a tumor-specific immune response. Examples of tumor antigens include, for example, MAGE, MART-1/Melan-A, gp100, Dipeptidyl peptidase IV (DPPIV), adenosine deaminase-binding protein (ADAbp), cyclophilin b, Colorectal associated antigen C017-1A/GA733, Carcinoembryonic Antigen (CEA) and its immunogenic epitopes CAP-1 and CAP-2, etv6, aml1, Prostate Specific Antigen (PSA) and its immunogenic epitopes PSA-1, PSA-2, and PSA-3, prostate-specific membrane antigen (PSMA), T-cell receptor/CD3-zeta chain, MAGE-family of tumor antigens (e. g., MAGE-A1, MAGE-A2, MAGE-A3, MAGE-A4, MAGE-A5, MAGE-A6, MAGE-A7, MAGE- A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE- C5), GAGE-family of tumor antigens (e.g., GAGE-1, GAGE-2, GAGE-3, GAGE-4, GAGE-5, GAGE-6, GAGE-7, GAGE-8, GAGE-9), BAGE, RAGE, LAGE-1, NAG, GnT-V, MUM-1, CDK4, tyrosinase, p53, MUC family, HER2/neu, p21ras, RCAS1, α-fetoprotein, E-cadherin, α-catenin, β-catenin and γ-catenin, p120ctn, gp100 Pmel117, PRAME, NY-ESO-1, cdc27, adenomatous polyposis coli protein (APC), fodrin, Connexin 37, Ig-idiotype, p15, gp75, GM2 and GD2 gangliosides, viral products such as human papilloma virus proteins, Smad family of tumor antigens, lmp-1, P1A, EBV-encoded nuclear antigen (EBNA)-1, brain glycogen phosphorylase, SSX-1, SSX-2 (HOM-MEL-40), SSX-1, SSX-4, SSX-5, SCP-1 and CT-7, and c-erbB-2; or an antigenic fragment of any of the foregoing.

Cancers or tumors associated with specific tumor antigens include, for example, acute lymphoblastic leukemia (etv6; aml1; cyclophilin b), B cell lymphoma (Ig-idiotype), glioma (E-cadherin; α-catenin; β-catenin; γ-catenin; p120ctn), bladder cancer (p21ras), biliary cancer (p21ras), breast cancer (MUC family; HER2/neu; c-erbB-2), cervical carcinoma (p53; p21ras), colon carcinoma (p21ras; HER2/neu; c-erbB-2; MUC family), colorectal cancer (C017-1A/GA733; APC), choriocarcinoma (CEA), epithelial cell-cancer (cyclophilin b), gastric cancer (HER2/neu; c-erbB-2; ga733 glycoprotein), hepatocellular cancer (α-fetoprotein), Hodgkins lymphoma (lmp-1; EBNA-1), lung cancer (CEA; MAGE-3; NY-ESO-1), lymphoid cell-derived leukemia (cyclophilin b), melanoma (p15 protein, gp75, oncofetal antigen, GM2 and GD2 gangliosides), myeloma (MUC family; p21ras), non-small cell lung carcinoma (HER2/neu; c-erbB-2), nasopharyngeal cancer (lmp-1; EBNA-1), ovarian cancer (MUC family; HER2/neu; c-erbB-2), prostate cancer (Prostate Specific Antigen (PSA) and its immunogenic epitopes PSA-1, PSA- 2, and PSA-3; PSMA; HER2/neu; c-erbB-2), pancreatic cancer (p21ras; MUC family; HER2/neu; c-erbB-2; ga733 glycoprotein), renal (HER2/neu; c-erbB-2), squamous cell cancers of cervix and esophagus (viral products such as human papilloma virus proteins), testicular cancer (NY-ESO-1), T cell leukemia (HTLV-1 epitopes), and melanoma (Melan-A/MART-1; cdc27; MAGE-3; p21ras; gp100 Pmel117).

Other cancer antigens that can be used together with the IRM compounds are provided in U.S. Patent Publication No. US 2002/0156033.
Methods for providing prophylactic and/or therapeutic treatments for certain cancers are described, for example, in U.S. Patent Publication Nos. US 2003/0161797; US 2004/0091491; US 2004/0175336; US 2004/0192585; and US 2005/0054665.

An immunostimulatory composition may include a substantially homogeneous population of antigenic peptide or, alternatively, may include two or more different antigenic peptides. In the case of an immunostimulatory composition that includes antigenic aggregate, the aggregates themselves may include two or more different antigenic peptides.

In some cases, a single antigenic peptide may be capable of inducing several distinct antigen-specific immune responses against several different antigenic portions of an antigenic peptide. For example, a full-length protein may contain several epitopes - i.e., many different amino acid segments, each of which is capable of inducing a distinct antigen-specific immune response. Consequently, even an immunostimulatory composition that includes a substantially homogeneous population of a single antigenic peptide may be capable of generating more than one antigen-specific immune response - i.e., immune responses directed against more than one epitope.

Providing two or more antigenic peptides in the reaction solution to react with the IRM compound, may produce a multi-antigen aggregate

An immunostimulatory composition that includes two or more different antigenic peptides may be desirable when, for example, more than one known antigen is associated with a particular condition. Such a composition may increase the efficacy of a treatment that includes administering the composition by, for example, generating an antigen-specific immune response against each of the antigens in the composition. In this way, affected cells may be "double targeted" and, as a result, may be more likely to be recognized by the immune system and/or may be cleared more rapidly.

An immunostimulatory composition that includes two or more antigenic peptides may also be desirable when, for example, an immune response is desired against a target that is capable of changing is immunogenic profile. For example, one challenge in developing an efficacious vaccine against certain viral conditions is that the antigenic portion of the virus that is the vaccine target can mutate so that the immune response induced by the vaccine no longer recognizes its target and, therefore, no longer recognizes the virus. An immunostimulatory composition that includes more than one antigenic peptide can generate an immune response against multiple antigenic targets on the virus. Thus, even if one target antigen mutates so that the immune response induced by that component of the vaccine no longer recognizes its target, the immune responses generated by another antigenic peptide may still recognize its target and, therefore, still recognize the virus.

The IRM moiety may be, or be derived from, any suitable IRM compound. IRM compounds can possess potent immunomodulating activity including but not limited to antiviral and antitumor activity. Certain IRMs modulate the production and secretion of cytokines. For example, certain IRM compounds induce the production and secretion of cytokines such as, e.g., Type I interferons, TNF-α, IL-1, IL-6, IL-8, IL-10, IL-12, MIP-1, and/or MCP-1. As another example, certain IRM compounds can inhibit production and secretion of certain T_{H}2 cytokines, such as IL-4 and IL-5. Additionally, some IRM compounds are said to suppress IL-1 and TNF (U.S. Patent No. 6,518,265).

Certain IRMs are small organic molecules (e.g., molecular weight under about 1000 Daltons, preferably under about 500 Daltons, as opposed to large biological molecules such as proteins, peptides, nucleic acids, and the like) such as those disclosed in, for example, U.S. Patent Nos. 4,689,338; 4,929,624; 5,266,575; 5,268,376; 5,346,905; 5,352,784; 5,389,640; 5,446,153; 5,482,936; 5,756,747; 6,110,929; 6,194,425; 6,331,539; 6,376,669; 6,451,810; 6,525,064; 6,541,485; 6,545,016; 6,545,017; 6,573,273; 6,656,938; 6,660,735; 6,660,747; 6,664,260; 6,664,264; 6,664,265; 6,667,312; 6,670,372; 6,677,347; 6,677,348; 6,677,349; 6,683,088; 6,756,382; 6,797,718; and 6,818,650; U.S. Patent Publication Nos. 2004/0091491; 2004/0147543; and 2004/0176367; and International Publication Nos. WO 2005/18551, WO 2005/18556, WO 2005/20999, WO 2005/032484, WO 2005/048933, WO 2005/048945, WO-2005/051317, WO 2005/051324, WO 2005/066169, WO 2005/066170, WO 2005/066172, WO 2005/076783, WO 2005/079195, WO 2005/123079, WO 2005/123080, WO 2006/009826, WO 2006/026760, WO 2006/028451, WO 2006/028545, WO 2006/028962, WO 2006/029115, and WO 2006/038923.

Additional examples of small molecule IRMs include certain purine derivatives (such as those described in U.S. Patent Nos. 6,376,501, and 6,028,076), certain imidazoquinoline amide derivatives (such as those described in U.S. Patent No. 6,069,149), certain imidazopyridine derivatives (such as those described in U.S. Patent No. 6,518,265), certain benzimidazole derivatives (such as those described in U.S. Patent 6,387,938), certain derivatives of a 4-aminopyrimidine fused to a five membered nitrogen containing heterocyclic ring (such as adenine derivatives described in U. S. Patent Nos. 6,376,501; 6,028,076 and 6,329,381; and in WO 02/08905), certain 3-β-D-ribofuranosylthiazolo[4,5-d]pyrimidine derivatives (such as those described in U.S. Publication No. 2003/0199461), and certain small molecule immuno-potentiator compounds such as those described, for example, in U.S. Patent Publication No. 2005/0136065.

Other IRMs include large biological molecules such as oligonucleotide sequences. Some IRM oligonucleotide sequences contain cytosine-guanine dinucleotides (CpG) and are described, for example, in U.S. Patent Nos. 6,194,388; 6,207,646; 6,239,116; 6,339,068; and 6,406,705. Some CpG-containing oligonucleotides can include synthetic immunomodulatory structural motifs such as those described, for example, in U.S. Patent Nos. 6,426,334 and 6,476,000. Other IRM nucleotide sequences lack CpG sequences and are described, for example, in International Patent Publication No. WO 00/75304. Still other IRM nucleotide sequences include guanosine- and uridine-rich single-stranded RNA (ssRNA) such as those described, for example, in Heil et al., Science, vol. 303, pp. 1526-1529, March 5, 2004.

Other IRMs include biological molecules such as aminoalkyl glucosaminide phosphates (AGPs) and are described, for example, in U.S. Patent Nos. 6,113,918; 6,303,347; 6,525,028; and 6,649,172.

In some embodiments of the present invention, the IRM compound may be an agonist of at least one TLR, preferably an agonist of TLR6, TLR7, or TLR8. The IRM may also in some cases be an agonist of TLR 9. In some embodiments of the present invention, the IRM compound may be a small molecule immune response modifier (e.g., molecular weight of less than about 1000 Daltons).

In some embodiments, the IRM compound may include a 2-aminopyridine fused to a five membered nitrogen-containing heterocyclic ring, or a 4-aminopyrimidine fused to a five membered nitrogen-containing heterocyclic ring.

Certain IRM compounds suitable for use in the invention include compounds having a 2-aminopyridine fused to a five membered nitrogen-containing heterocyclic ring. Such compounds include, for example, imidazoquinoline amines including but not limited to substituted imidazoquinoline amines such as, for example, amide substituted imidazoquinoline amines, sulfonamide substituted imidazoquinoline amines, urea substituted imidazoquinoline amines, alkoxyl substituted imidazoquinoline amines, aryl ether substituted imidazoquinoline amines, heterocyclic ether substituted imidazoquinoline amines, amido ether substituted imidazoquinoline amines, sulfonamido ether substituted imidazoquinoline amines, urea substituted imidazoquinoline ethers, thioether substituted imidazoquinoline amines, hydroxylamine substituted imidazoquinoline amines, oxime substituted imidazoquinoline amines, 6-, 7-, 8-, or 9-aryl, heteroaryl, nitrogen-containing heterocyclyl, aryloxy or arylalkyleneoxy substituted imidazoquinoline amines, and imidazoquinoline diamines; tetrahydroimidazoquinoline amines including but not limited to amide substituted tetrahydroimidazoquinoline amines, sulfonamide substituted tetrahydroimidazoquinoline amines, urea substituted tetrahydroimidazoquinoline amines, alkoxy substituted tetrahydroimidazoquinoline amines, aryl ether substituted tetrahydroimidazoquinoline amines, heterocyclic ether substituted tetrahydroimidazoquinoline amines, amido ether substituted tetrahydroimidazoquinoline amines, sulfonamido ether substituted tetrahydroimidazoquinoline amines, urea substituted tetrahydroimidazoquinoline ethers, thioether substituted tetrahydroimidazoquinoline amines, hydroxylamine substituted tetrahydroimidazoquinoline amines, oxime substituted tetrahydroimidazoquinoline amines, and tetrahydroimidazoquinoline diamines; imidazopyridine amines including but not limited to amide substituted imidazopyridine amines, sulfonamide substituted imidazopyridine amines, urea substituted imidazopyridine amines, alkoxy substituted imidazopyridine amines, aryl ether substituted imidazopyridine amines, heterocyclic ether substituted imidazopyridine amines, amido ether substituted imidazopyridine amines, sulfonamido ether substituted imidazopyridine amines, urea substituted imidazopyridine ethers, and thioether substituted imidazopyridine amines; 1,2-bridged imidazoquinoline amines; 6,7-fused cycloalkylimidazopyridine amines; imidazonaphthyridine amines; tetrahydroimidazonaphthyridine amines; oxazoloquinoline amines; thiazoloquinoline amines; oxazolopyridine amines; thiazolopyridine amines; oxazolonaphthyridine amines; thiazolonaphthyridine amines; pyrazolopyridine amines; pyrazoloquinoline amines; tetrahydropyrazoloquinoline amines; pyrazolonaphthyridine amines; tetrahydropyrazolonaphthyridine amines; and 1*H*-imidazo dimers fused to pyridine amines, quinoline amines, tetrahydroquinoline amines, naphthyridine amines, or tetrahydronaphthyridine amines.

In certain embodiments, the IRM compound may be an imidazonaphthyridine amine, a tetrahydroimidazonaphthyridine amine, an oxazoloquinoline amine, a thiazoloquinoline amine, an oxazolopyridine amine, a thiazolopyridine amine, an oxazolonaphthyridine amine, a thiazolonaphthyridine amine, a pyrazolopyridine amine, a pyrazoloquinoline amine, a tetrahydropyrazoloquinoline amine, a pyrazolonaphthyridine amine, or a tetrahydropyrazolonaphthyridine amine.

In certain embodiments, the IRM compound may be a substituted imidazoquinoline amine, a tetrahydroimidazoquinoline amine, an imidazopyridine amine, a 1,2-bridged imidazoquinoline amine, a 6,7-fused cycloalkylimidazopyridine amine, an imidazonaphthyridine amine, a tetrahydroimidazonaphthyridine amine, an oxazoloquinoline amine, a thiazoloquinoline amine, an oxazolopyridine amine, a thiazolopyridine amine, an oxazolonaphthyridine amine, a thiazolonaphthyridine amine, a pyrazolopyridine amine, a pyrazoloquinoline amine, a tetrahydropyrazoloquinoline amine, a pyrazolonaphthyridine amine; or a tetrahydropyrazolonaphthyridine amine.

As used herein, a substituted imidazoquinoline amine refers to an amide substituted imidazoquinoline amine, a sulfonamide substituted imidazoquinoline amine, a urea substituted imidazoquinoline amine, an aryl ether substituted imidazoquinoline amine, a heterocyclic ether substituted imidazoquinoline amine, an amido ether substituted imidazoquinoline amine, a sulfonamido ether substituted imidazoquinoline amine, a urea substituted imidazoquinoline ether, a thioether substituted imidazoquinoline amine, a hydroxylamine substituted imidazoquinoline amine, an oxime substituted imidazoquinoline amine, a 6-, 7-, 8-, or 9-aryl, heteroaryl, aryloxy or arylalkyleneoxy substituted imidazoquinoline amine, or an imidazoquinoline diamine. As used herein, substituted imidazoquinoline amines specifically and expressly exclude 1-(2-methylpropyl)-1*H*-imidazo[4,5-*c*]quinolin-4-amine and 4-amino-α,α-dimethyl-2-ethoxymethyl-1*H*-imidazo[4,5-*c*]quinolin-1-ethanol.

Suitable IRM compounds also may include the purine derivatives, imidazoquinoline amide derivatives, benzimidazole derivatives, adenine derivatives, aminoalkyl glucosaminide phosphates, and oligonucleotide sequences described above. In some embodiments, the IRM compound may be a compound identified as an agonist of one or more TLRs.

In one particular embodiment, the immune response modifier moiety of the compound is derived from *N*-[6-({2-[4-amino-2-(ethoxymethyl)-1*H*-imidazo[4,5-c]quinolin-1-yl]-1,1-dimethylethyl amino)-6-oxohexyl]-4-azido-2-hydroxybenzamide.

In another embodiment, the immune response modifier moiety of the compound is derived from *N*-{2-[4-amino-2-(ethoxymethyl)-1H-imidazo[4,5-c]quinolin-1-yl]-1,1-dimethylethyl}-6-[(3-mercaptopropanoyl)amino]hexanamide.

In another embodiment, the immune response modifier moiety of the compound is derived from *N*-{2-[2-(ethoxymethyl)-1*H*-imidazo[4,5-*c*]quinolin-1-yl]-1,1-dimethylethyl}-6-[(3-mercaptopropanoyl)amino]hexanamide.

Also described herein are methods of inducing an antigen-specific immune response in a subject. Generally, the method includes contacting immune cells with an immunostimulatory composition that includes either a partially purified antigenic aggregate or a partially purified monomeric conjugate in an amount effective to cause the immune cells to generate an immune response against an antigen of the immunostimulatory composition

Also described herein is a method of treating a condition in a subject. Generally, the method includes administering to a subject an immunostimulatory composition that includes a partially purified antigenic aggregate in an amount effective to ameliorate at least one symptom or at least one clinical sign of the condition. The method may be used to provide a prophylactic treatment, a therapeutic treatment, or both. As used herein, a prophylactic treatment refers to a treatment that limits, to any extent, the development and/or appearance of a symptom or clinical sign of a condition. As used herein, a therapeutic treatment refers to a treatment that ameliorates one or more existing symptoms or clinical signs associated with a condition.

The immunostimulatory composition may be provided in any formulation suitable for administration to a subject. Suitable types of formulations are described, for example, in U.S. Pat. No. 5,238,944; U.S. Pat. No. 5,939,090; U.S. Pat. No. 6,245,776; European Patent No. EP 0 394 026; and U.S. Patent Publication No. 2003/0199538. The compound may be provided in any suitable form including but not limited to a solution, a suspension, an emulsion, or any form of mixture. The compound may be delivered in formulation with any pharmaceutically acceptable excipient, carrier, or vehicle. For example, the formulation may be delivered in a conventional topical dosage form such as, for example, a cream, an ointment, an aerosol formulation, a non-aerosol spray, a gel, a lotion, and the like. The formulation may further include one or more additives including but not limited to adjuvants, lipids, oils, mucosal adhesives, skin penetration enhancers, colorants, fragrances, flavorings, moisturizers, thickeners, and the like. Finally, the immunostimulatory composition may be formulated to provide timed release or a depot effect - i.e., may include molecules of the immunostimulatory composition attached to a support material, incorporated into a lipid membrane, lipid vesicle, or liposome, or composed within a biodegradable polymer matrix.

A formulation may be administered in any suitable manner such as, for example, non-parenterally or parenterally. As used herein, non-parenterally refers to administration through the digestive tract, including by oral ingestion. Parenterally refers to administration other than through the digestive tract such as, for example, intravenously, intramuscularly, transdermally, subcutaneously, transmucosally (e.g., by inhalation), or topically.

The suitable composition of a formulation will vary according to factors known in the art including but not limited to the physical and chemical nature of the immunostimulatory composition, the nature of the carrier, the intended dosing regimen, the state of the subject's immune system (e.g., suppressed, compromised, stimulated), the method of administering the immunostimulatory composition, and the species to which the formulation is being administered. Accordingly, it is not practical to set forth generally the composition of a formulation effective for all possible applications. Those of ordinary skill in the art, however, can readily determine an appropriate formulation with due consideration of such factors.

In some cases, the methods described herein include administering an immunostimulatory composition to a subject in a formulation of, for example, from about 0.0001% to about 10% (unless otherwise indicated, all percentages provided herein are weight/weight with respect to the total formulation) to the subject, although in some embodiments the immunostimulatory composition may be administered at a concentration outside of this range. In certain cases, the method includes administering to a subject a formulation that includes from about 0.01% to about 1% immunostimulatory composition, for example, a formulation that includes about 0.1 % to about 0.5% immunostimulatory composition.

An effective amount of an immunostimulatory composition is an amount sufficient to induce an antigen-specific immune response. The precise amount of immunostimulatory composition necessary to induce an antigen-specific immune response will vary according to factors known in the art including but not limited to the physical and chemical nature of the immunostimulatory composition, the nature of the carrier, the intended dosing regimen, the state of the subject's immune system (e.g., suppressed, compromised, stimulated), the inherent immunogenicity of the antigen, the potency of the IRM moiety, the method of administering the immunostimulatory composition, and the species to which the formulation is being administered. Accordingly, it is not practical to set forth generally the amount that constitutes an amount of immunostimulatory composition effective for inducing an antigen-specific immune response for all possible applications. Those of ordinary skill in the art, however, can readily determine the appropriate amount with due consideration of such factors.

In some cases, the methods described herein include administering sufficient immunostimulatory composition to provide a dose of, for example, from about 1 ng/kg to about 50 mg/kg to the subject, although in some embodiments the methods may be performed by administering immunostimulatory composition in a dose outside this range. In some of these cases, the method includes administering sufficient immunostimulatory composition to provide a dose of from about 100 ng/kg to about 10 mg/kg to the subject, for example, a dose of from about 100 µg/kg to about 5 mg/kg. In one particular embodiment, the method includes administering from about 1 mg/kg to about 2.5 mg/kg of immunostimulatory composition to the subject.

Alternatively, the dose may be calculated using actual body weight obtained just prior to the beginning of a treatment course. For the dosages calculated in this way, body surface area (m²) is calculated prior to the beginning of the treatment course using the Dubois method: m² = (wt kg^{0.425} x height cm^{0.725}) x 0.007184.

In some cases, the methods described herein may include administering sufficient immunostimulatory composition to provide a dose of, for example, from about 0.01 mg/m² to about 10 mg/m².

The dosing regimen may depend at least in part on many factors known in the art including but not limited to the physical and chemical nature of the immunostimulatory composition, the nature of the carrier, the amount of immunostimulatory composition being administered, the state of the subject's immune system (e.g., suppressed, compromised, stimulated), the inherent immunogenicity of the antigen, the potency of the IRM moiety, the method of administering the immunostimulatory composition, and the species to which the formulation is being administered. Accordingly it is not practical to set forth generally the dosing regimen effective for use in all possible applications. Those of ordinary skill in the art, however, can readily determine an appropriate dosing regimen with due consideration of such factors.

In some instances, the immunostimulatory composition may be administered, for example, from a single administration to about multiple administrations per week. In certain cases, the immunostimulatory composition may be administered from once (i.e., a single administration) to about once per day. In particular cases, the immunostimulatory composition is administered once. In other cases, the immunostimulatory composition is administered twice (prime and boost). In yet other cases, the immunostimulatory composition is administered once initially (prime), then administered again at regular or irregular intervals, as needed (boost).

Conditions that may be treated by administering an immunostimulatory composition include, but are not limited to:
(a) viral diseases such as, for example, diseases resulting from infection by an adenovirus, a herpesvirus (e.g., HSV-I, HSV-II, CMV, or VZV), a poxvirus (e.g., an orthopoxvirus such as variola or vaccinia, or molluscum contagiosum), a picomavirus (e.g., rhinovirus or enterovirus), an orthomyxovirus (e.g., influenzavirus); a paramyxovirus (e.g., parainfluenzavirus, mumps virus, measles virus, and respiratory syncytial virus (RSV)), a coronavirus (e.g., SARS), a papovavirus (e.g., papillomaviruses, such as those that cause genital warts, common warts, or plantar warts), a hepadnavirus (e.g., hepatitis B virus), a flavivirus (e.g., hepatitis C virus or Dengue virus), or a retrovirus (e.g., a lentivirus such as HIV);
(b) bacterial diseases such as, for example, diseases resulting from infection by bacteria of, for example, the genus Escherichia, Enterobacter, Salmonella, Staphylococcus, Shigella, Listeria, Aerobacter, Helicobacter, Klebsiella, Proteus, Pseudomonas, Streptococcus, Chlamydia, Mycoplasma, Pneumococcus, Neisseria, Clostridium, Bacillus, Corynebacterium, Mycobacterium, Campylobacter, Vibrio, Serratia, Providencia, Chromobacterium, Brucella, Yersinia, Haemophilus, or Bordetella;
(c) other infectious diseases, such chlamydia, fungal diseases including but not limited to candidiasis, aspergillosis, histoplasmosis, cryptococcal meningitis, or parasitic diseases including but not limited to malaria, pneumocystis carnii pneumonia, leishmaniasis, cryptosporidiosis, toxoplasmosis, and trypanosome infection;
(d) neoplastic diseases, such as intraepithelial neoplasias, cervical dysplasia, actinic keratosis, basal cell carcinoma, squamous cell carcinoma, renal cell carcinoma, Kaposi's sarcoma, melanoma, solid tumor neoplasias such as, for example, breast cancer, lung cancer, and colon cancer, leukemias including but not limited to myelogeous leukemia, chronic lymphocytic leukemia, multiple myeloma, non-Hodgkin's lymphoma, cutaneous T-cell lymphoma, B-cell lymphoma, and hairy cell leukemia, and other cancers;
(e) T_{H}2-mediated, atopic diseases, such as atopic dermatitis or eczema, eosinophilia, asthma, allergy, allergic rhinitis, and Ommen's syndrome;
(f) certain autoimmune diseases such as systemic lupus erythematosus, essential thrombocythaemia, multiple sclerosis, discoid lupus, alopecia areata; and
(g) diseases associated with wound repair such as, for example, inhibition of keloid formation and other types of scarring (e.g., enhancing wound healing, including chronic wounds).

Certain immunostimulatory compositions may be particularly helpful in individuals having compromised immune function. For example, certain immunostimulatory compositions may be used for treating the opportunistic infections and tumors that occur after suppression of cell mediated immunity in, for example, transplant patients, cancer patients and HIV patients.

The methods described herein may be performed on any suitable subject. Suitable subjects include but are not limited to animals such as but not limited to humans, non-human primates, poultry, fowl, rodents, dogs, cats, horses, pigs, sheep, goats, or cows.

### Examples

The following examples have been selected merely to further illustrate features, advantages, and other details of the invention.

### IRM Compounds

The IRM compounds used in the examples are shown in Table 1.

**Table 1**

| Compound | Chemical Name | Reference |
|---|---|---|
| IRM1 | *N*-[6-({2-[4-amino-2-(ethoxymethyl)-1*H*-imidazo[4,5-*c*]quinolin-1-yl]-1,1-dimethylethyl}amino)-6-oxohexyl]-4-azido-2-hydroxybenzamide | U.S. 2004/0091491 IRM1 |
| IRM2 | *N*-{2-[4-amino-2-(ethoxymethyl)-1H-imidazo[4,5-c]quinolin-1-yl]-1,1-dimethylethyl}-6-[(3-mercaptopropanoyl)amino]hexanamide | |
| IRM3 | *N*-{2-[2-(ethoxymethyl)-1*H*-imidazo[4,5-*c*]quinolin-1-yl]-1,1-dimethylethyl}-6-[(3-mercaptopropanoyl)amino]hexanamide | |

### Preparation of IRM2: N-{2-[4-amino-2-(ethoxymethyl)-1H-imidazo[4,5-c]quinolin-1-yl]-1,1-dimethylethyl} -6-[(3 -mercaptopropanoyl)amino]hexanamide

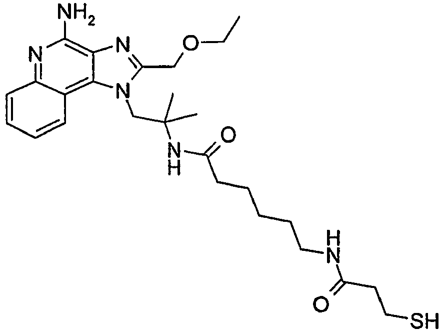

### Part A

To a solution of 6-(carbobenzyloxyamino) caproic acid (8.49 grams (g), 32.0 millimole (mmol)) in DMF (50 mL) at 0°C was added *N*-hydroxysuccinimide and 1-(3-dimethylaminopropyl)-3-ethylcarbodiimide hydrochloride (EDC). After a short period, the solution was added to a 0°C solution of 1-(2-amino-2-methylpropyl)-2-(ethoxymethyl)-1*H*-imidazo[4,5-*c*]quinolin-4-amine (prepared as described in U.S. Patent Publication No. 2004/0091491, 10 g, 32 mmol) in DMF (100 mL). The mixture was allowed to warm to room temperature and was stirred for 3 days. The solution was diluted with water (400 mL) and extracted with ethyl acetate (3x). The organic layers were combined and washed with water (2x) and brine. The organic layer was dried over sodium sulfate, filtered, and concentrated. The crude product was purified by HPFC on silica gel three times (gradient elution with CMA in chloroform) to provide 4.90 g of benzyl 6-({2-[4-amino-2-(ethoxymethyl)-1*H*-imidazo[4,5-*c*]quinolin-1-yl]-1,1-dimethylethyl}amino)-6-oxohexylcarbamate as a white foam.

### Part B

A mixture of benzyl 6-({2-[4-amino-2-(ethoxymethyl)-1*H*-imidazo[4,5-*c*]quinolin-1-yl]-1,1-dimethylethyl}amino)-6-oxohexylcarbamate (4.90 g, 8.75 mmol) and 10% palladium on carbon (0.5 g) in ethanol (100 mL) was hydrogenated on a Parr apparatus at 20-40 psi (1.4 x 10⁵ - 2.8 x 10⁵ Pa) for 1 day, during which time fresh hydrogen was introduced several times. The mixture was filtered through CELITE filter agent. The filtrate was concentrated under reduced pressure to yield a white foam that was used directly in the next step.

### Part C

To a mixture of 3,3'-dithiodipropionic acid (920 mg, 4.38 mmol) and 1-hydroxybenzotriazole (HOBT) (1.42 g, 10.5 mmol) in dimethylformamide (DMF) (50 mL) at 0°C was added 1-(3-dimethylaminopropyl)-3-ethylcarbodiimide hydrochloride (1.85 g, 9.63 mmol). The mixture was stirred at 0°C for 4 hours. The material from Part B (8.75 mmol) was dissolved in DMF (20 mL), cooled to 0°C, and the cold solution of the activated diacid was added in one portion, with two DMF rinses (10 mL each). The reaction was allowed to warm slowly to room temperature overnight. Several more portions of EDC were added to the reaction at 0°C over the next two days. The reaction was allowed to stir at room temperature for several days more, then was diluted with water and saturated aqueous sodium bicarbonate and was extracted with ethyl acetate several times. The combined organic extracts were washed with water and brine, and were concentrated under reduced pressure. The crude product was purified by HPFC to give 3.8 g of the disulfide dimer of *N*-{2-[4-amino-2-(ethoxymethyl)-1*H*-imidazo[4,5-c]quinolin-1-yl]-1,1-dimethylethyl} -6-[(3 -mercaptopropanoyl)amino]hexanamide.

### Part D

The material from Part C (3.8 g, 3.7 mmol) was dissolved in methanol (30 mL) at room temperature. Tris(2-carboxyethyl)phosphine hydrochloride (1.38 g, 4.81 mmol) was added, followed by water (3 mL), and 12.5 M aqueous sodium hydroxide (1.12 mL, 14.1 mmol). The solution was stirred at room temperature for 2 hours and then was cooled to 0°C. The solution was adjusted to pH 6 with 1 M aqueous hydrochloric acid (approximately 14 mL). The methanol was removed under reduced pressure and aqueous sodium bicarbonate was added. The mixture was extracted with dichloromethane (3 x). The organic extracts were combined, washed with water and brine, dried over sodium sulfate, filtered, and concentrated under reduced pressure. The crude product was purified by HPFC on silica gel (gradient elution with 0-50% CMA in chloroform). The appropriate fractions were concentrated to provide 2.36 g of *N*-{2-[4-amino-2-(ethoxymethyl)-1*H*-imidazo[4,5-c]quinolin-1-yl]-1,1-dimethylethyl}-6-[(3-mercaptopropanoyl)amino]hexanamide.
White foam, MS (ESI) m/z 515 (M + H)⁺. ¹H NMR (300 MHz, CDCl₃) δ 8.22 (dd, 1H), 7.80 (dd, 1H), 7.51 (ddd, 1H), 7.32 (ddd, 1H), 5.96 (m, 1H), 5.59 (s, 1H), 5.51 (br s, 2H), 5.06 (s, 2H), 4.84 (br s, 2H), 3.62 (q, *J* = 6.9 Hz, 2H), 3.25 (q, *J* = 6.9 Hz, 2H), 2.81 (m, 2H), 2.50 (t, *J* = 6.9 Hz, 2H), 1.98 (m, 2H), 1.61-1.18 (m, 13H), 1.24 (t, *J* = 6.9 Hz, 3H). Anal. calcd for C₂₆H₃₈N₆O₃S•0.5H₂O: C, 59.63; H, 7.51; N, 16.05; S, 6.12. Found: C, 59.89; H, 7.66; N, 16.22; S, 6.25.

### Preparation of IRM3: N-{2-[2-(ethoxymethyl)-1H-imidazo[4,5-c]quinolin-1-yl]-1,1-dimethylethyl}-6-[(3-mercaptopropanoyl)amino]hexanamide

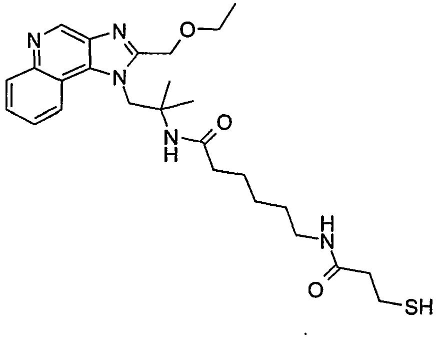

### Part A

6-[(*tert*-Butoxycarbonyl)amino]hexanoic acid (6.81 g, 29.5 mmol), 1-hydroxybenzotriazole (3.98 g, 29.5 mmol), and 1-(3-dimethylaminopropyl)-3-ethylcarbodiimide hydrochloride (EDC) (5.66 g, 29.5 mmol) were added to a 0°C solution of 2-[2-(ethoxymethyl)-1*H*-imidazo[4,5-*c*]quinolin-1-yl]-1,1-dimethylethylamine (which was prepared by acid-mediated deprotection of *tert*-butyl 2-[2-(ethoxymethyl)-1*H-*imidazo[4,5-*c*]quinolin-1-yl]-1,1-dimethylethylcarbamate, which is described in U.S. Patent Publication No. 2004/0091491, 8.00 g, 26.8 mmol) in DMF (80 mL). The solution was stirred at room temperature overnight. More 6-[(*tert*-butoxycarbonyl)amino]hexanoic acid and EDC were added and the solution was stirred for an additional hour. The solution was partitioned between aqueous sodium bicarbonate and ethyl acetate. The aqueous layer was extracted with ethyl acetate (2x). The organic layers were combined, washed with water and brine, dried over sodium sulfate, filtered, and concentrated under reduced pressure. The crude product was purified by chromatography on silica gel to provide 3.99 g of *tert*-butyl 6-({2-[2-(ethoxymethyl)-1*H*-imidazo[4,5-*c*]quinolin-1-yl]-1,1-dimethylethyl}amino)-6-oxohexylcarbamate as a foam.

### Part B

Trifluoroacetic acid (30 mL) was added slowly to a solution of *tert*-butyl 6-({2-[2-(ethoxymethyl)-1*H*-imidazo[4,5-*c*]quinolin-1-yl]-1,1-dimethylethyl}amino)-6-oxohexylcarbamate (3.99 g, 7.82 mmol) in dichloromethane (80 mL) at room temperature. After 1.5 hours, the solution was concentrated under pressure to afford an oil. The oil was dissolved in a small amount of water and concentrated ammonium hydroxide. The resulting basic mixture was extracted with dichloromethane multiple times. The combined organic extracts were dried over sodium sulfate, filtered, and concentrated under reduced pressure to afford 3.4 g of 6-amino-*N*-{2-[2-(ethoxymethyl)-1*H*-imidazo[4,5-*c*]quinolin-1-yl]-1,1-dimethylethyl}hexanamide, which was used in the next step without further purification.

### Part C

A solution of 6-amino-*N*-{2-[2-(ethoxymethyl)-1*H*-imidazo[4,5-*c*]quinolin-1-yl]-1,1-dimethylethyl}hexanamide (1.35 g, 3.28 mmol), 3,3'-dithiodipropionic acid (0.345 g, 1.64 mmol), 1-hydroxybenzotriazole (0.443 g, 3.28 mmol), and 1-(3-dimethylaminopropyl)-3-ethylcarbodiimide hydrochloroide (0.692, 3.61 mmol) in DMF (10 mL) was stirred at room temperature overnight. The solution was concentrated under reduced pressure and partitioned between saturated aqueous sodium bicarbonate and ethyl acetate/methanol. The organic layers were combined, dried over sodium sulfate, filtered, and concentrated under reduced pressure. The crude product was purified by HPFC on silica gel (gradient elution with CMA/chloroform) to provide the disulfide dimer of *N*-{2-[2-(ethoxymethyl)-1*H*-imidazo[4,5-*c*]quinolin-1-yl]-1,1-dimethylethyl}-6-[(3-mercaptopropanoyl)amino]hexanamide.

### Part D

*N*-{2-[2-(Ethoxymethyl)-1*H*-imidazo[4,5-*c*]quinolin-1-yl]-1,1-dimethylethyl}-6-[(3-mercaptopropanoyl)amino]hexanamide (2.14 g, 2.15 mmol) was dissolved in methanol (20 mL). Tris(2-carboxyethyl)phosphine (0.800 g, 2.79 mmol), water (2 mL), and 12.5 M NaOH (0.65 mL, 8.17 mmol) were added. The solution was allowed to stir for 1.5 hours at room temperature, then was cooled in an ice bath. The solution was adjusted to pH 6 with 1 M HCl and the resulting mixture was concentrated under reduced pressure to remove the methanol. The mixture was partitioned between saturated aqueous sodium bicarbonate and dichloromethane. The aqueous layer was extracted multiple times with dichloromethane. The organic phases were combined, washed with water and brine, dried over sodium sulfate, filtered, and concentrated under reduced pressure to provide 1.69 g of *N*-{2-[2-(ethoxymethyl)-1*H*-imidazo[4,5-*c*]quinolin-1-yl]-1,1-dimethylethyl}-6-[(3-mercaptopropanoyl)amino]hexanamide as a white foam that was heated under vacuum to produce a glassy solid.
Colorless glassy solid. MS (ESI) m/z 500 (M + H)⁺. ¹H NMR (300 MHz, CDCl₃) δ 9.30 (s, 1H), 8.52 (m, 1H), 8.26 (m, 1H), 7.72-7.62 (m, 2H), 5.84 (m, 1H), 5.59 (br s, 1H), 5.18 (br s, 2H), 4.91 (br s, 2H) 3.63 (m, 2H), 3.27 (m, 2H), 2,81 (m, 2H), 2.49 (t, *J* = 6.9 Hz, 2H), 2.05 (t, *J* = 7.5 Hz, 2H), 1.63-1.22 (m, 16H). Anal. calcd for C₂₆H₃₇N₅O₃S: C, 62.50; H, 7.46; N, 14.02; S, 6.42. Found: C, 62.23; H, 7.54; N, 13.90; S, 6.65.

### Example 1

IRM1 was suspended in dimethyl sulfoxide (DMSO) to 10 milligrams per milliliter (mg/mL). Ovalbumin (OVA, Sigma Chemical Co., St. Louis, MO) was suspended in phosphate buffered saline (PBS) to 10 mg/mL and the pH adjusted to > 10.0 by the addition of NaOH. Two IRM1/OVA reaction mixtures were prepared. First, 900 microliters (µL) of the ovalbumin solution was mixed with 100 µL of the IRM1 solution in a single well of a 12-well tissue culture plate, generating an approximate 4:1 molar excess of IRM1. Second, 800 µL of the OVA solution was mixed with 100 µL of the IRM1 solution in a single well of a 12-well tissue culture plate, generating an approximate 8:1 molar excess of IRM1. The plates were placed on ice and a long wavelength UV light source was placed directly over the plates as close to the wells containing the IRM1/OVA mixtures as possible. The mixtures were irradiated for 15 minutes. The resulting conjugation reaction mixtures were removed from the wells and resuspended in PBS to a final concentration of 4.5 mg/mL ovalbumin

Separately, ovalbumin solution (900 µL + 100 µL DMSO) without IRM was irradiated as described above.

### Example 2

The 4:1 IRM1 + OVA conjugation mixture, the 8:1 IRM1 + OVA conjugation mixture, and the OVA irradiated in the absence of IRM1 from Example 1 were run separately on a System Gold HPLC (Beckman Coulter, Inc., Fullerton, CA). Each mixture was run on a Superdex 200 10/300 GL size exclusion column (Amersham Biosciences, Piscataway, NJ) at a flow rate of 0.5 mL per minute (mL/min) using a 1X PBS, pH 7.4 running buffer. The 1X PBS, pH 7.4 was made from a 10X PBS solution (Cat.# P313-300, Biosource International, Inc., Carmarillo, CA) diluted 1:10 in Milli-Q purified water. Data was collected using a detection wavelength of 280 nanometers (nM) with 8 data points per second reported on the chromatograms. Fractions were collected in one-minute increments for the duration of the HPLC run.

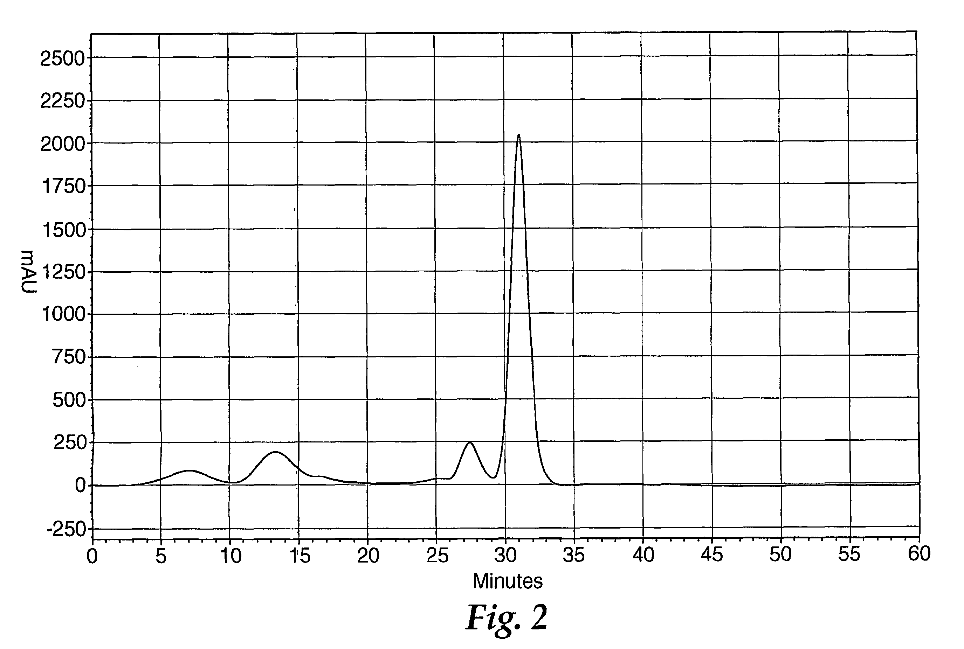

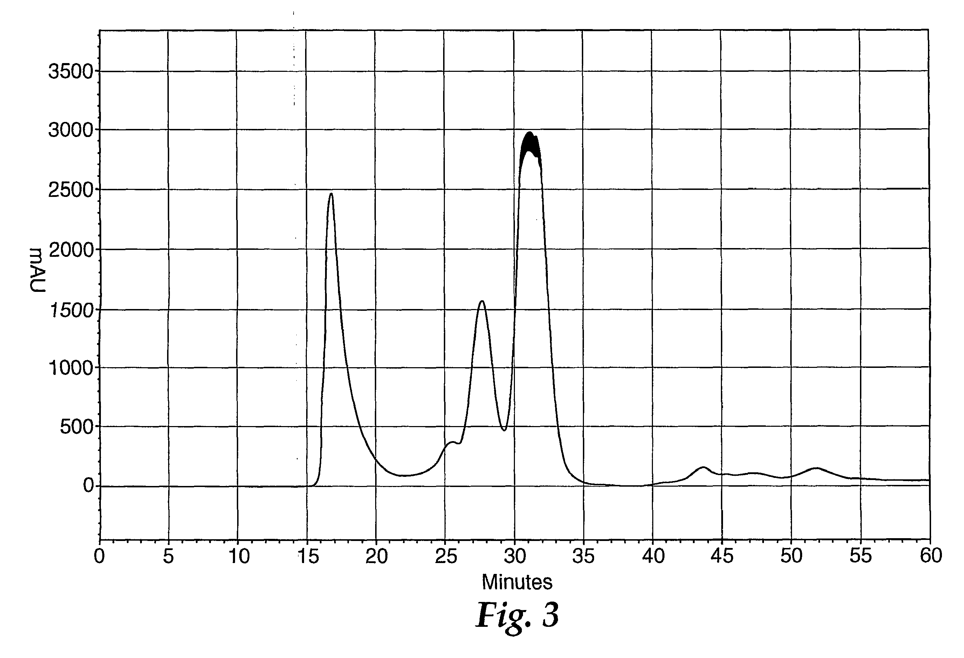

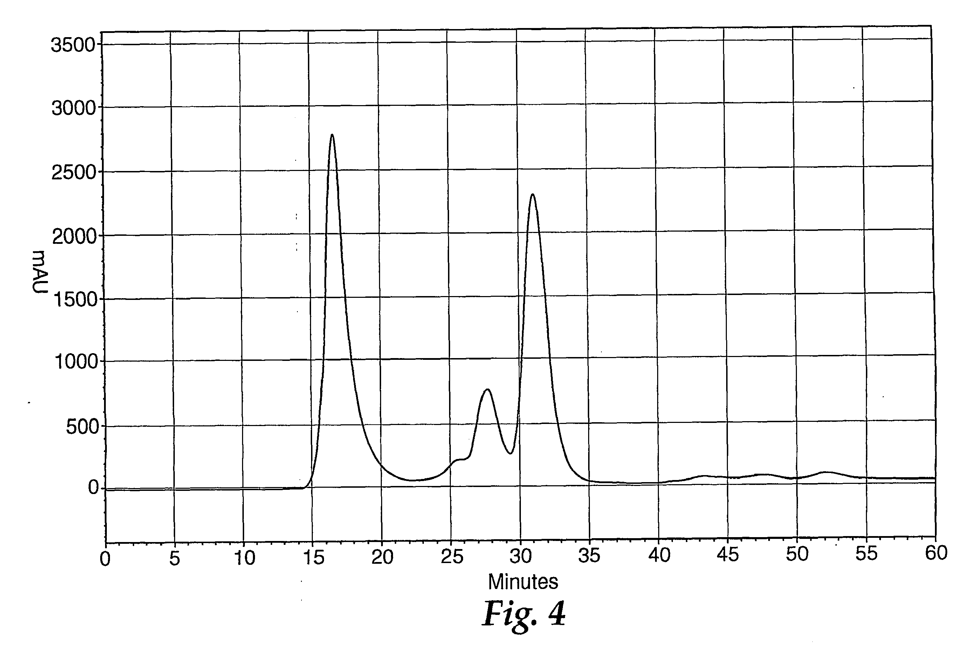

The chromatogram of the product of the 4:1 IRM1 + OVA conjugation reaction is shown in Fig. 3. The chromatogram of the product of the 8:1 IRM1 + OVA conjugation reaction is shown in Fig. 4. The chromatogram of the product of the UV irradiated OVA in the absence of IRM is shown in Fig. 2.

### Example 3

Irradiated (+UV) or non-irradiated (-UV) samples of ovalbumin OVA with or without IRM1 (±IRM) were prepared as described in Example 1. Half of each sample was run on a HPLC as described in Example 2. The Fractions 16-20 or fractions 25-34 from the HPLC runs were pooled together. A micro BCA protein assay kit (Cat#23225, Pierce Biotechnology, Inc., Rockford, IL) was used to quantify the amount of OVA protein of all the samples. 2x10⁶ chicken OVA-specific OT-I⁺/GFP⁺ lymphocytes per mouse were adoptively transferred into syngeneic C57BL/6 mice (Charles River Laboratories, Wilmington, MA).

Two days later the mice were immunized intra-peritoneally with 100 micrograms (µg) of the pre-HPLC OVA+IRM1+UV (UV irradiated OVA/IRM1 mixture), OVA+IRM1-UV (non-irradiated OVA/IRM1 mixture), or OVA+UV (UV irradiated ovalbumin); 93 µg of the pooled fractions 25-34 of OVA+IRM1+UV, OVA+IRM1-UV, or OVA+UV; 44 µg of the pooled fractions 16-20; or were untreated.

Three days after immunization, mice were sacrificed and spleens were removed. The total number of splenocytes was determined by using a Guava PCA 96 (Guava-Technologies, Inc., Hayward, CA). Splenocytes were stained with a propidium iodine (PI)-labeled mouse anti-CD8⁺ antibody (BD Biosciences Pharmingen, San Diego, CA) and the percent OT-I⁺/GFP⁺ lymphocytes was determined by flow cytometry gating on PI-CD8⁺/GFP⁺ lymphocytes. The total number of OT-I⁺GFP⁺ lymphocytes was determined by multiplying the total number of splenocytes by the percent OT-I⁺/GFP⁺ lymphocytes.

Results are shown in Fig. 1.

### Example 4

Cationic bovine serum albumin (cBSA) was made using BSA from Calbiochem (Cat# 12657, San Diego, CA) using the method described in Border, W., et al., Journal of Clinical Investigation, Vol. 69, pgs. 451-461, (1982). An IRM1 + cBSA conjugate was made by mixing a 10 mg/mL cBSA protein solution and a 10 mg/mL IRM1 solution using the method described in Example 1.

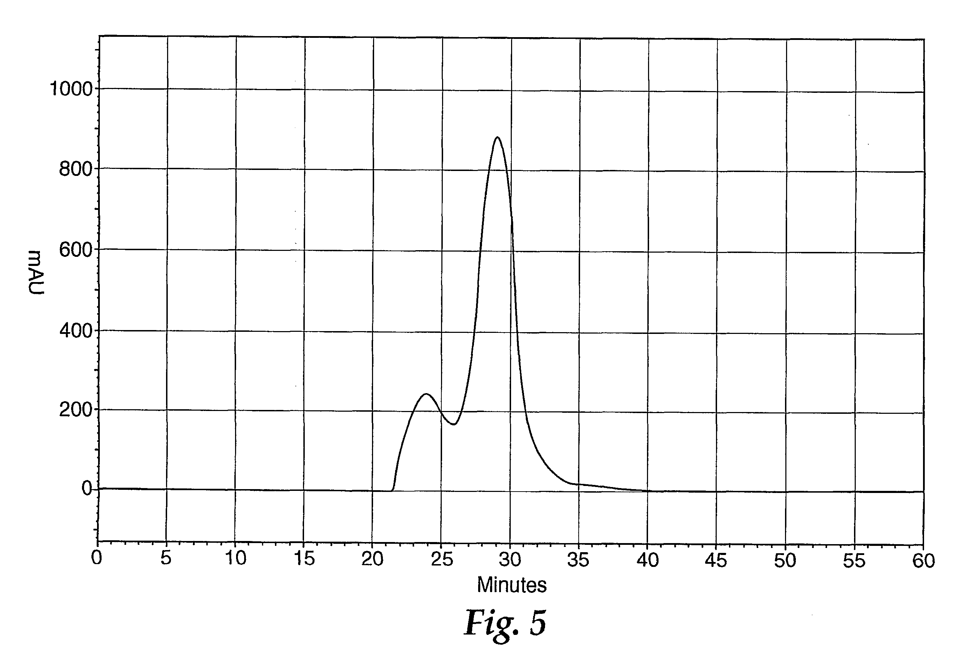

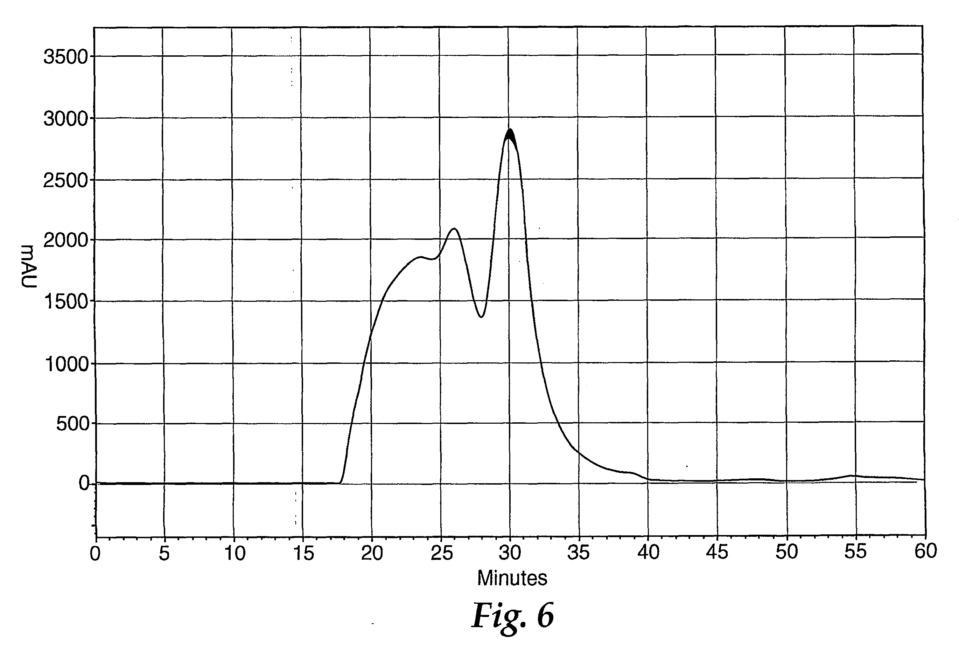

The cBSA/IRM1 conjugate was then run on HPLC using the same conditions described in Example 2. The chromatogram of the product of the IRM1 + cBSA conjugation reaction is shown in Fig. 6. A chromatogram of the product of UV irradiated cBSA in the absence of IRM1 is shown in Fig. 5.

### Example 5

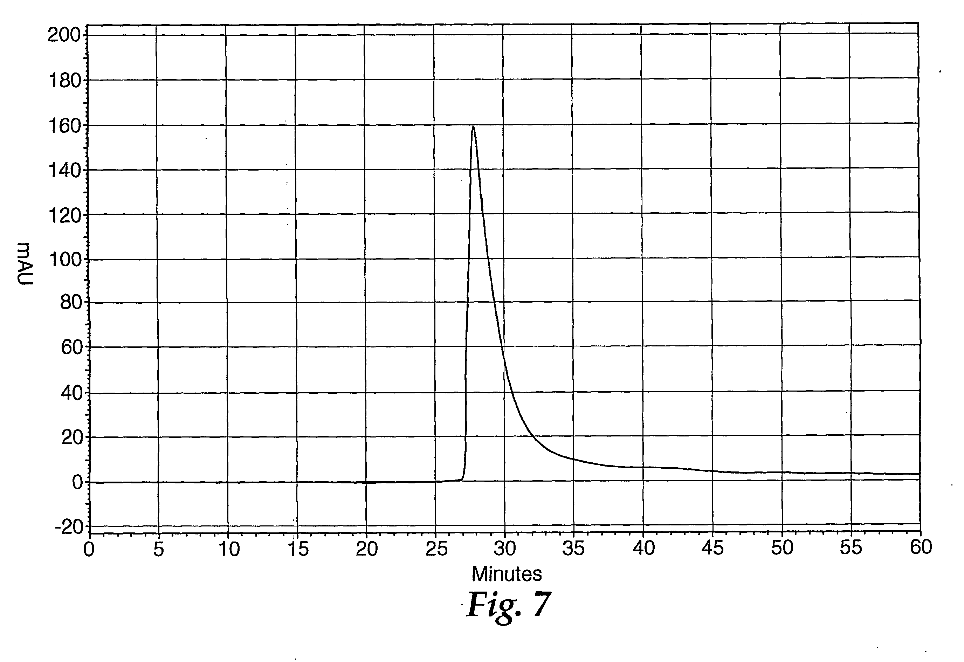

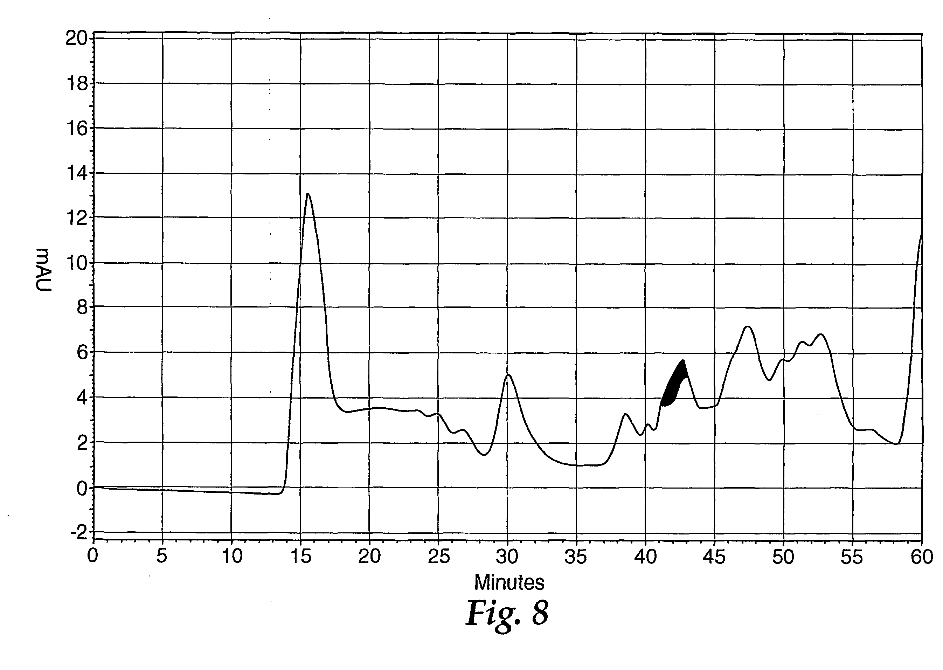

IRM1 and HIV Gag41 protein were conjugated using the method described in Example 1 with a 4:1 molar excess of IRM1. Chromatograms of the IRM1 + HIV Gag41 conjugated mixture and an IRM1 + HIV Gag41 unconjugated mixture were generated using the method described in Example 2. The chromatogram of the product of the unconjugated IRM1 + HIV Gag41 is shown in Fig. 7. The chromatogram of the product of the IRM1 + HIV Gag41 conjugation reaction is shown in Fig. 8.

### Example 6

### Modification of Hen Egg Lysomzyme Antigen:

Hen Egg Lysozyme (HEL; Sigma) was adjusted to a concentration of 14.8 mg/mL in PBS, pH 7.4. A NHS-PEO₈-Maleimide (Pierce) crosslinker was dissolved in DMSO to a concentration of 5 mg/mL. The crosslinker solution was slowly added to the HEL solution at the desired molar excess while mixing, and incubated for one hour at room temperature. The modified HEL was purified by applying the mixture to a PD 10 desalting column equilibrated with PBS, pH 7.2 containing 5 mM EDTA. One-milliliter fractions were collected and the fractions containing modified IgG, determined by measuring the absorbance at a wavelength of 280 nm, were pooled together.

Conjugation of HEL to IRMs

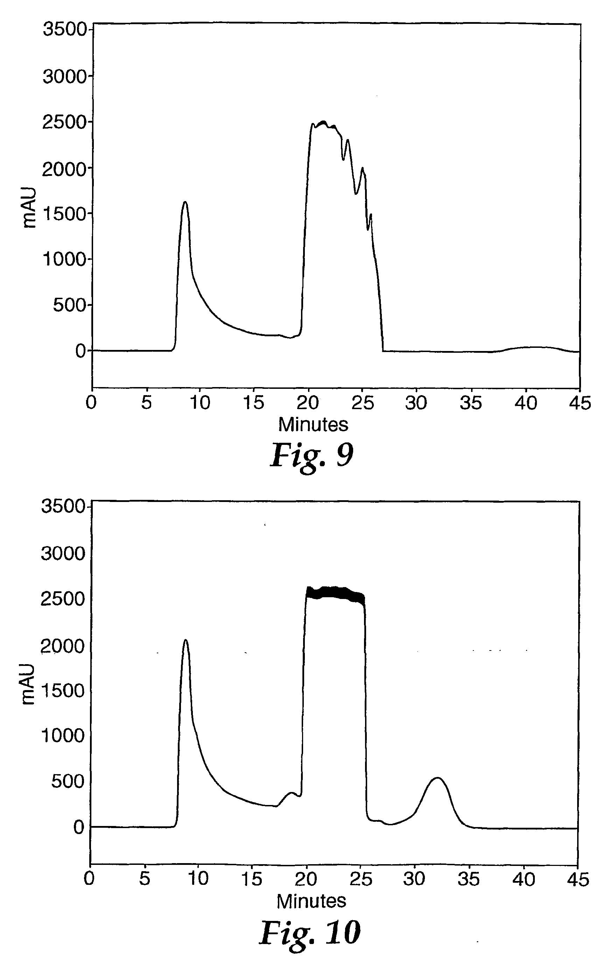

IRM2 or IRM3 was dissolved in DMSO at a concentration of 10 mM. IRM 2 or IRM3 were added to the modified HEL, as prepared above, at a four-fold molar excess of IRM. L-cysteine was added to the modified HEL, as prepared above, at a five-fold molar excess of L-cysteine to the amount of crosslinker used to modify the HEL. The mixture was incubated overnight at room temperature. To quench the IRM reactions, a 500 mM L-cysteine solution (dissolved in 1 M Tris, pH 8.0) was added at 0.01 times (volume/volume) the reaction mixture. The samples were run on HPLC as described in Example 2. The chromatograms of the IRM2-HEL, IRM3-HEL, and L-cys-HEL are shown in Fig. 9-11, respectively.

The IRM- HEL conjugates were purified by size exclusion chromatography using PBS, pH 7.4 as the column running buffer, at a flow rate of 1 mL/min. One-milliliter fractions of the mixtures were collected and measured at 280 nm. Fractions containing the monomeric or aggregate IRM-HEL conjugates were pooled and filtered under sterile conditions through a 0.2-micron filter. The concentrations of the IRM-HEL conjugates were determined by BCA assay using unmodified HEL as a standard. The filtered conjugate was stored at 4 °C for future testing in biological assays.

### Example 7

Four to six week old BALB/c mice were injected subcutaneously in each footpad with HEL alone (5 or 50 µg) or 5 µg of either aggregate or monomer fractions from the IRM-HEL conjugates generated in Example 6. Ten days after injection, mice were sacrificed and blood was collected via cardiac puncture. Blood was allowed to clot at room temperature (RT) for 30 minutes, centrifuged at 2,500 RPM at 4°C, collected and stored at -30°C for future HEL antibody analysis.

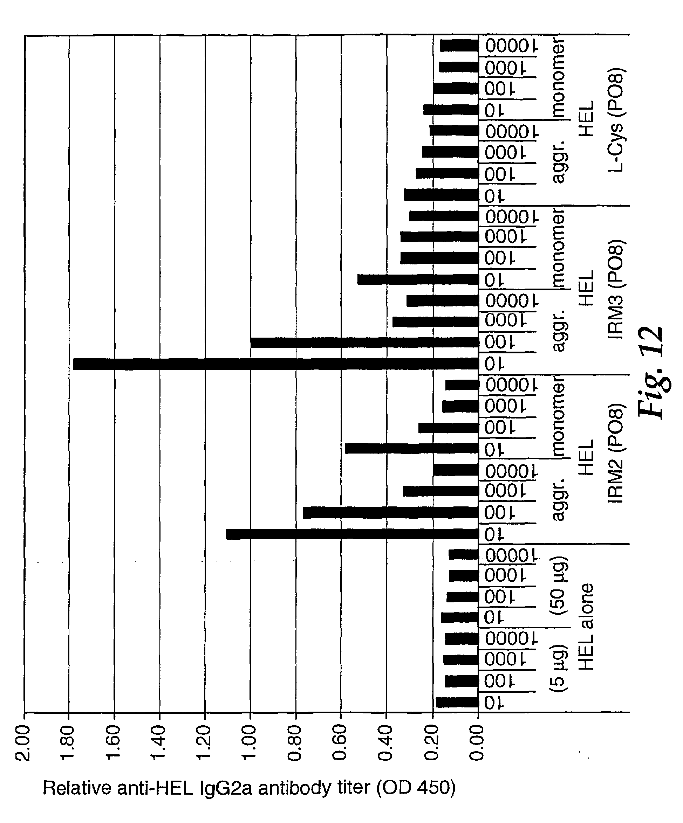

A mouse anti-HEL IgG2a-antibody ELISA was performed on the serum. 96-well plates (Nunc MAXISORP; Nunc, Rochester, NY) were coated with 100 µL/well of 0.020 mg/mL HEL in coating buffer (0.05 M sodium bicarbonate, 0.02% sodium azide, pH 9.6) overnight at 4°C. Plates were then washed with wash buffer (0.05 M sodium bicarbonate, 0.02% sodium azide, pH 9.6) and saturated with 200 µL per well of blocking buffer (0.025% powdered milk, 0.003 M HEPES, 0.4 M sodium chloride, 3.8mM disodium EDTA). After washing three times, 100 µL of 10-fold dilutions (1:10 to 1:10,000) of serum samples in blocking buffer were added to plates and incubated for one hour at RT. Plates were then washed three times and 100 µL/well of horseradish peroxidase labeled goat anti-mouse IgG2a (Serotec, Raleigh, NC), 1:4000 dilution in blocking buffer, was added to the plates and incubated for one hour at RT. Plates were washed two times and 100 µL/well of TMB substrate solution (BD Biosciences Pharmigen) was added. Plates were incubated for approximately 8 minutes in the dark at RT and then 100 µL/well of stop solution was added. Plates were read immediately at an absorbance of 450 nm with a reference wavelength of 570 nm. Results are shown in Fig. 12.

### Example 8

### Part A

A 25 mg/mL OVA solution was prepared in a reaction buffer. The reaction buffer was a 0.5 M PBS solution containing 0.15 M NaCl and 2 mM EDTA prepared using the manufactures instructions for the Sigma Kit PBS-1. Two OVA control mixtures were prepared by transferring 0.5 mL aliquots of the OVA solution into a 1.5 mL Eppendorf tube and treated with 10 µL of DMSO (Control 1, Cl) or 10 µL of a 37 mg/mL solution of N-ethylmaleimide (NMI; Pierce) in DMSO (Control 2, C2). Additional sample mixtures were prepared by transferring 1.0 mL aliquots of the OVA solution into a 1.5 mL Eppendorf tube and treated with 20 µL of the NMI solution. The mixtures were incubated for 2 to 2.5 hours at room temperature with gentle mixing. Each mixture was purified over a PD-10 desalting column equilibrated with the reaction buffer, using an absorbance of 280 nM. Fractions were collected, the fractions containing protein were pooled together and the absorbance (280 nM) of the pooled fractions was recorded to determine the final concentration of the modified OVA protein. The concentration of protein in the Control solutions and OVA solutions were 7.5 mg/mL and 12mg/ml, respectively.

### Part B

A 0.1237 M solution of sulfosuccinimidyl 4-(*N*-maleimidomethyl)cyclohexane-1-carboxylate (Sulfo-SMCC; Pierce) was prepared in DMSO. The purified OVA solutions from Part A were aliquoted and mixed with 5-fold (5X), 10-fold (10X), 20-fold (20X), or 60-fold (60X) molar excess of Sulfo-SMCC to OVA. The mixtures were incubated for 1.5 to 2 hours at room temperature with gentle mixing. Each mixture was purified over a PD-10 desalting column and fractions containing protein were pooled and analyzed as described above.

### Part C

Solutions of 0.124 M IRM1 and 0.124 M L-cysteine (Pierce) were made in DMSO. Purified 5X, 10X, 20X, and 60X solutions from Part B were mixed with 12-fold, 17-fold, 27-fold, and 40-fold molar excess of IRM1 to protein, respectively. Purified 10X (10X Cys), 20X (20X Cys), and Control 2 from above were mixed with 17-fold, 27-fold, and 10-fold excess of cysteine to protein, respectively. A 1.5 mL volume of Control 1 was mixed with 40 µL of DMSO. All mixtures were incubated for 45 minutes at room temperature except for the mixtures containing cysteine, which went directly into a 4°C refrigerator. All samples remained at 4°C until purification the next day. Each mixture was purified over a PD-10 desalting column and fractions containing protein were pooled and analyzed as described above. Samples were kept at 4°C until biological testing was performed.

### In Vivo Testing

3x10⁶ chicken ovalbumin-specific OT-I⁺/GFP⁺ lymphocytes per mouse were adoptively transferred into syngeneic C57BL/6 mice (Charles River Laboratories, Wilmington, MA).

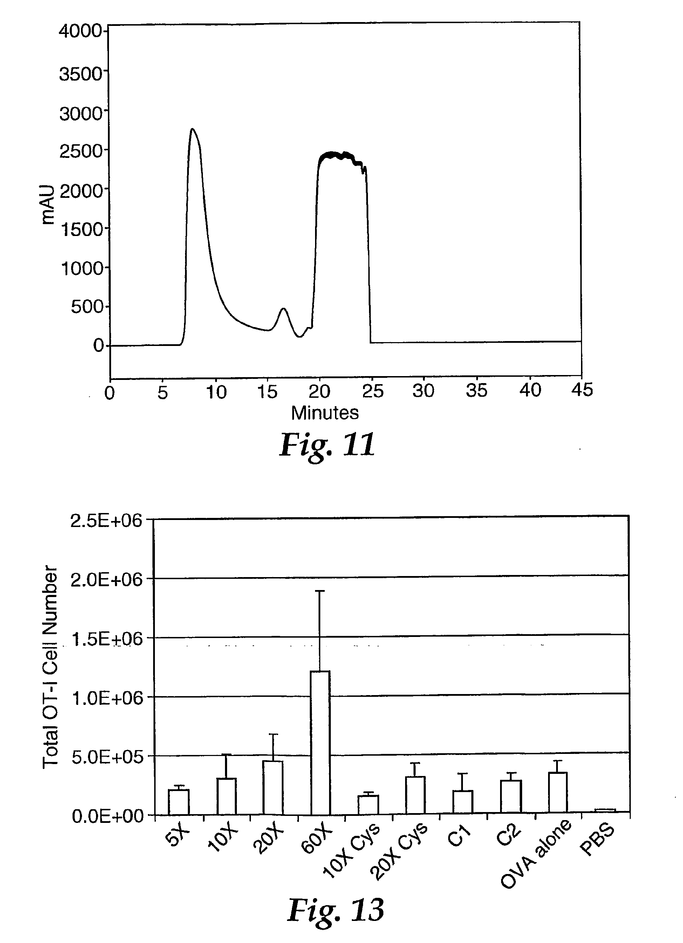

Twenty-four hours later, the mice were immunized subcutaneously in the rear flank with 100 µL (containing 100 µg of OVA, determined by BCA protein assay, in OVA containing samples) of each of the 5X, 10X, 20X, 60X, 10X Cys, 20X Cys, Control 1 (C1), Control 2 (C2), OVA alone, or PBS alone. Four days after immunization, mice were sacrificed and.the inguinal lymph nodes were removed and homogenized into a single cell suspension. The total number of lymphocytes was determined by using a Guava PCA 96 (Guava Technologies, Inc., Hayward, CA). Lymphocytes were stained with a propidium iodine (PI)-labeled mouse anti-CD8⁺ antibody (BD Biosciences Pharmingen, San Diego, CA) and the percent OT-I⁺/GFP⁺ lymphocytes was determined by flow cytometry gating on PI-CD8⁺/GFP⁺ lymphocytes. The total number of OT-I⁺/GFP⁺ lymphocytes was determined by multiplying the total number of lymphocytes by the percent OT-I⁺/GFP⁺ lymphocytes. Results are shown in Figure 13.

### Example 9

Bovine Serum Albumin (BSA; Calbiochem) was dissolved in PBS, pH 7.4 at a concentration of 15 mg/mL. To remove residual BSA dimer and aggregates prior to conjugation, the BSA was purified by size exclusion chromatography using PBS, pH 7.4 as the column running buffer at a flow rate of 1 mL/min. Fractions containing monomeric BSA were pooled and used as the starting material for conjugation (6.38 mg/mL in PBS, pH 7.4). To 1 mL aliquots (9.6 X 10-5 mMoles) of monomeric BSA, a 20- or 40-fold molar excess of Sulfo-SMCC, prepared at 10mg/mL in DMSO, was slowly added to the BSA while mixing. The mixtures were incubated at room temperature for 1 hour.

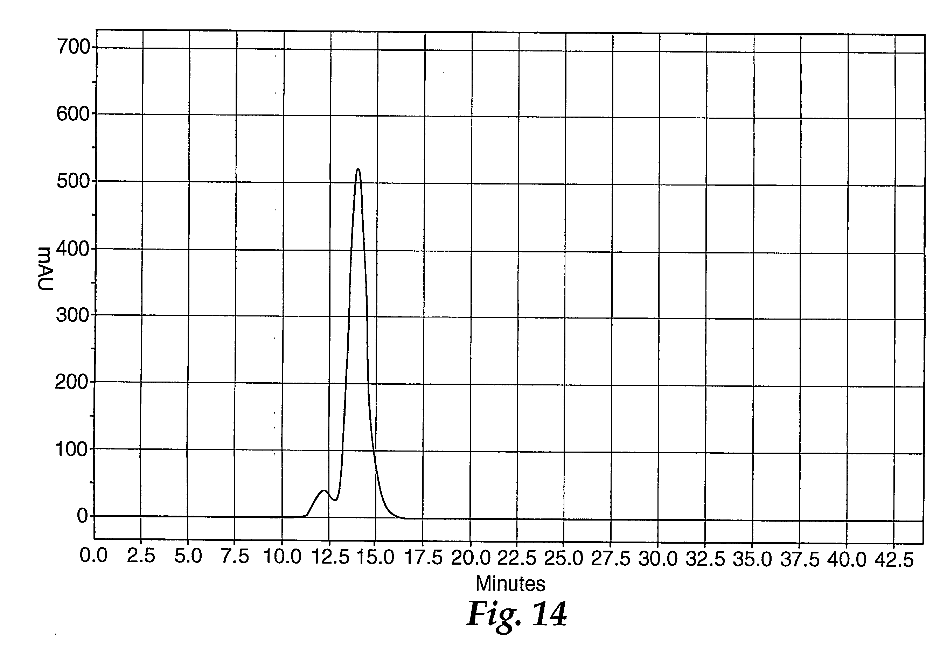

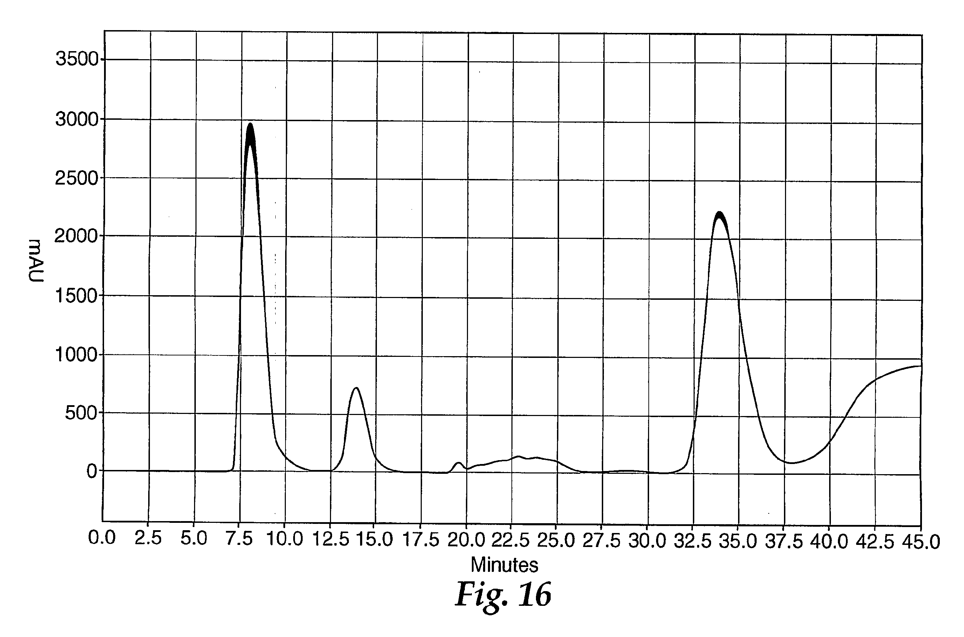

In the second step of the reaction, the maleimide activated BSA was split into 2 equal aliquots and a 60- or 120-fold excess of either IRM2 or IRM3 (3-fold excess to Sulfo-SMCC used in first step), prepared at 10 mg/mL in DMSO, was added to the activated BSA. Following overnight incubation at room temperature, 5uL of 500mM L-Cysteine HCl, prepared in 1N NaOH, was added to each conjugate for 30 minutes to quench unreacted maleimide and then purified by size exclusion chromatography, as described above. Fractions containing either monomeric conjugate or aggregated conjugate were pooled, sterile filtered and protein concentrations determined using the BCA assay kit. The chromatogram of the unconjugated BSA is shown in Fig. 14. The chromatogram of the product of the 20X and 40X Sulfo-SMCC IRM2 + BSA conjugation reaction is shown in Fig. 15 and 16, respectively. The chromatogram of the product of the 20X and 40X Sulfo-SMCC IRM3 + BSA conjugation reaction is shown in Fig. 17 and 18, respectively.

Illustrative embodiments and examples are provided as examples only and are not intended to limit the scope of the present invention. The scope of the invention is limited only by the claims set forth as follows.

## Claims

1. An immunostimulatory composition consisting essentially of:
an at least partially purified antigenic aggregate that comprises at least one immune response modifier (IRM) moiety and at least two antigenic peptides, in which at least one antigenic peptide is covalently coupled to the immune response modifier moiety.

2. The immunostimulatory composition of claim 1 wherein the aggregate comprises at least two different peptides.

3. The immunostimulatory composition of claim 2 comprising at least two different IRM/antigen conjugates, wherein a first immune response modifier/antigen conjugate comprises a first antigenic peptide covalently coupled to an immune response modifier moiety and a second immune response modifier/antigen conjugate comprises a second antigenic peptide covalently coupled to an immune response modifier moiety.

4. The immunostimulatory composition of claim 1-3 wherein at least one antigenic peptide is at least an antigenic portion of a viral antigen.

5. The immunostimulatory composition of claim 4 wherein the viral antigen is an HIV antigen.

6. The immunostimulatory composition of claim 5 wherein the HIV antigen is at least an antigenic portion of Gag41.

7. A method of making an immunostimulatory composition, the method comprising:
(i) providing a mixture of immune response modifier compound and antigenic peptides;
(ii) causing antigenic aggregates comprising at least one immune response modifier moiety and at least two antigenic peptides, in which at least one antigenic peptide is covalently coupled to an immune response modifier moiety, to form from the mixture, thereby also defining a non-aggregated portion of the mixture; and
(iii) separating at least a portion of the antigenic aggregates of (ii) from the monomeric IRM/antigen conjugates.

8. The method of claim 7 wherein the antigenic aggregates and the non-aggregated portion of the mixture are separated by high performance liquid chromatography.

## Patentansprüche

1. Immunstimulierende Zusammensetzung, bestehend im Wesentlichen aus:
einem wenigstens teilweise aufgereinigten antigenen Aggregat, das wenigstens eine IRM(Immune Response Modifier)-Gruppierung und wenigstens zwei antigene Peptide umfasst, wobei wenigstens ein antigenes Peptid kovalent an die IRM-Gruppierung gekoppelt ist.

2. Immunstimulierende Zusammensetzung nach Anspruch 1, wobei das Aggregat wenigstens zwei unterschiedliche Peptide umfasst.

3. Immunstimulierende Zusammensetzung nach Anspruch 2, umfassend wenigstens zwei unterschiedliche IRM/Antigen-Konjugate, wobei ein erstes IRM/Antigen-Konjugat ein erstes kovalent an eine IRM-Gruppierung gekoppeltes antigenes Peptid umfasst und ein zweites IRM/Antigen-Konjugat ein zweites kovalent an eine IRM-Gruppierung gekoppeltes antigenes Peptid umfasst.

4. Immunstimulierende Zusammensetzung nach Anspruch 1-3, wobei es sich bei wenigstens einem antigenen Peptid wenigstens um einen antigenen Anteil eines viralen Antigens handelt.

5. Immunstimulierende Zusammensetzung nach Anspruch 4, wobei es sich bei dem viralen Antigen um ein HIV-Antigen handelt.

6. Immunstimulierende Zusammensetzung nach Anspruch 5, wobei es sich bei dem HIV-Antigen wenigstens um einen antigenen Anteil von Gag41 handelt.

7. Verfahren zur Herstellung einer immunstimulierenden Zusammensetzung, bei dem man
(i) ein Gemisch von IRM-Verbindung und antigenen Peptiden bereitstellt;
(ii) die Bildung wenigstens eine IRM-Gruppierung und wenigstens zwei antigene Peptide, wobei wenigstens ein antigenes Peptid kovalent an eine IRM-Gruppierung gekoppelt ist, umfassender antigener Aggregate aus dem Gemisch veranlasst, wodurch auch ein nicht aggregierter Anteil des Gemischs definiert wird; und
(iii) die antigenen Aggregate aus (ii) wenigstens anteilweise von den monomeren IRM/Antigen-Konjugaten trennt.

8. Verfahren nach Anspruch 7, wobei die antigenen Aggregate und der nicht aggregierte Anteil des Gemischs über Hochleistungsflüssigkeitschromatographie getrennt werden.

## Revendications

1. Composition immunostimulante constituée essentiellement de :
un agrégat antigénique au moins partiellement purifié qui comprend au moins une fraction modificateur de la réponse immunitaire (MRI) et au moins deux peptides antigéniques, au moins un peptide antigénique étant couplé de façon covalente à la fraction modificateur de la réponse immunitaire.

2. Composition immunostimulante selon la revendication 1 dans laquelle l'agrégat comprend au moins deux peptides différents.

3. Composition immunostimulante selon la revendication 2 comprenant au moins deux conjugués MRI/antigène différents, dans laquelle un premier conjugué modificateur de la réponse immunitaire/antigène comprend un premier peptide antigénique couplé de façon covalente à une fraction modificateur de la réponse immunitaire et un second conjugué modificateur de la réponse immunitaire/antigène comprend un second peptide antigénique couplé de façon covalente à une fraction modificateur de la réponse immunitaire.

4. Composition immunostimulante selon la revendication 1-3 dans laquelle au moins un peptide antigénique est au moins une partie antigénique d'un antigène viral.

5. Composition immunostimulante selon la revendication 4 dans laquelle l'antigène viral est un antigène du VIH.

6. Composition immunostimulante selon la revendication 5 dans laquelle l'antigène du VIH est au moins une partie antigénique de Gag41.

7. Procédé de fabrication d'une composition immunostimulante, le procédé comprenant :
(i) l'obtention d'un mélange de composé modificateur de la réponse immunitaire et de peptides antigéniques ;
(ii) la provocation de la formation d'agrégats antigéniques comprenant au moins une fraction modificateur de la réponse immunitaire et au moins deux peptides antigéniques, dans lesquels au moins un peptide antigénique est couplé de façon covalente à une fraction modificateur de la réponse immunitaire, à partir du mélange, ce qui définit également une partie non agrégée du mélange ; et
(iii) la séparation d'au moins une partie des agrégats antigéniques de (ii) des conjugués MRI/antigène monomères.

8. Procédé selon la revendication 7 dans lequel les agrégats antigéniques et la partie non agrégée du mélange sont séparés par chromatographie liquide à haute performance.
